# EUROPEAN PATENT APPLICATION

(11) **EP 4 410 806 A1**
(43) Date of publication of application: **07.08.2024**
(21) Application number: 22876415.5
(22) Date of filing: 29.09.2022
(51) Int. Cl.: C07K 5/06, A61K 38/05, A61P 17/00, A61P 17/14, A61P 29/00, A61P 31/14, A61P 35/00, A61P 37/06, A61P 37/08, A61P 43/00, C07K 5/02, C07K 5/062, C12N 9/12

(54) **DEGRADATION INDUCER**

(30) Priority: 30.09.2021 JP 2021161316
(71) Applicant: ASKA Pharmaceutical Co., Ltd., Tokyo 108-8532 (JP)
(72) Inventor: KATO, Jun-ya, Fujisawa-shi, Kanagawa 251-8555 (JP); KORENAGA, Shigeru, Fujisawa-shi, Kanagawa 251-8555 (JP); IWAKURA, Masaru, Fujisawa-shi, Kanagawa 251-8555 (JP)
(74) Representative: Bardehle Pagenberg Partnerschaft mbB Patentanwälte Rechtsanwälte
(86) International application number: PCT/JP2022/036353
(87) International publication number: WO 2023/054549

(57) **Abstract**

To provide a novel compound that induces selective degradation of a non-receptor tyrosine kinase (and especially a tyrosine kinase that is part of the JAK family), or a pharmaceutically acceptable salt thereof, and others. Provided is a compound represented by formula I: 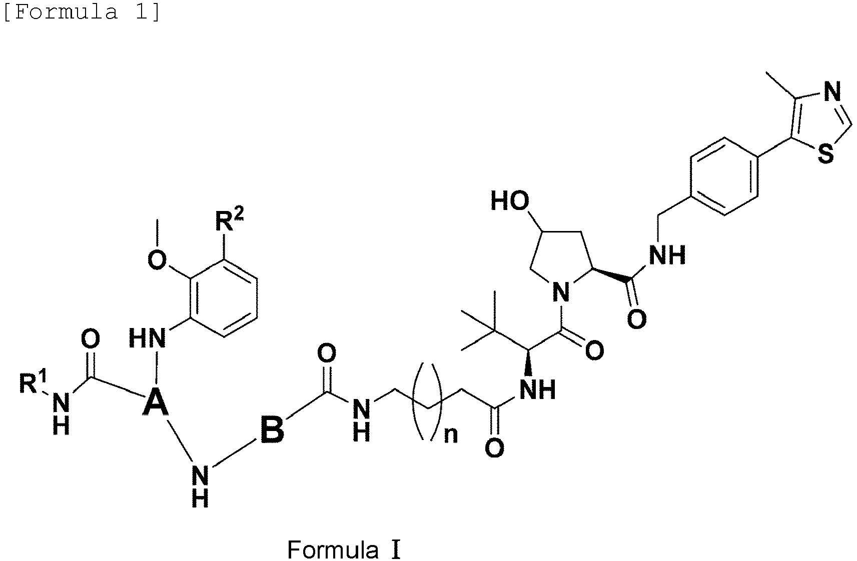 wherein
R¹ is a hydrogen atom or a C₁₋₃ alkyl group optionally substituted with one or more deuterium atoms;
R² is CONHR³ (wherein R³ is a C₁₋₃ alkyl group optionally substituted with one or more OH groups) or a triazole group optionally substituted with one or more C₁₋₃ alkyl groups;
A is a pyridyl group or a pyridazinyl group;
B is 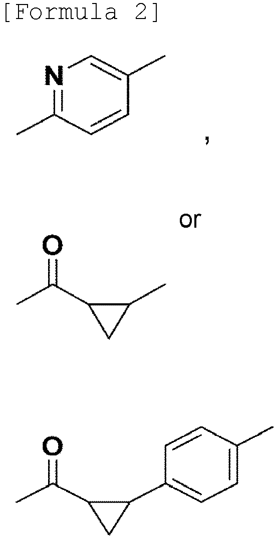 and
n is an integer between 0 and 12,
or a pharmaceutically acceptable salt or the like thereof.

## Description

### Technical Field

The present invention relates to a novel compound that induces degradation of non-receptor tyrosine kinases, or a pharmaceutically acceptable salt thereof. Furthermore, the present invention relates to a degrader (degradation inducer) or medicament containing such a compound or pharmaceutically acceptable salt thereof, and to a method of improving, preventing and/or treating a condition or disease requiring modulation, such as the suppression, of activation of cytokine signaling pathways by administering such medicament to a subject in need thereof.

### Cross-reference to Related Applications

The present application is based on Japanese Patent Application No. 2021-161316 filed on September 30, 2021, and claims the benefit of and priority to that Japanese Patent Application, the entirety of which is incorporated by reference herein.

### Background Art

Non-receptor tyrosine kinases, especially JAKs (Janus kinases), are known to play an important role in the JAK-STAT (Janus kinases-signal transducer and activator of transcription) pathway, which transmits signals of cytokines and the like.

JAKs are tyrosine kinases that bind to cytokine receptors which are the most upstream of the signaling pathway, and are activated when a cytokine binds to a receptor, thereby activating the corresponding signal transducer and activator of transcription (STAT). The activated STAT translocates to the nucleus and initiates gene transcription, which enables cytokine's function. The JAK family includes four subtypes, JAK1, JAK2, JAK3, and tyrosine kinase 2 (Tyk2), which are activated by various cytokines through a variety of JAK-STAT combinations (Non Patent Literature 1).

It is known that JAK1, JAK2, and Tyk2 are expressed in a wide range of cells while JAK3 is expressed almost exclusively in lymphocytes. It is further known that JAK3 is responsible for IL-2, 4, 7, 9, 15, and 21 signaling together with JAK1, and also that JAK2 is responsible for the signaling of, for example, IL-3 and 5, granulocyte macrophage colony-stimulating factor (GMCSF), erythropoietin, thrombopoietin, and growth hormones. In addition, it is known that JAK1 and JAK2 are involved in interferon-γ (IFN-γ) signaling, JAK1 and Tyk2 are involved in interferon-α/β (IFN-α/β) signaling, and JAK2 and Tyk2 are involved in IL-12 and 23 signaling. Furthermore, JAK1, JAK2, and Tyk2 are known to be involved in the signaling of IL-6, 10, 11, 13, 19, 20, 22 and 27, granulocyte colony stimulating factor (GCSF), LIF (leukemia inhibitory factor), and oncostatin M (OSM) (Non Patent Literatures 1 and 2).

Because of such importance of JAKs in the JAK-STAT pathway, research has been conducted for regulating cytokine signaling by inhibiting JAKs to suppress the occurrence of adverse events, such as symptoms and pathologies caused by various cytokines, in order to help in the treatment of autoimmune diseases, inflammatory diseases, and the like. Some small molecule JAK inhibitors have already been approved as therapeutic agents for rheumatoid arthritis and ulcerative colitis. Inhibition of JAK1 is also expected to have therapeutic effects on atopic dermatitis and alopecia. In addition, human genome-wide association study has shown that a preventive effect against a wide range of autoimmune diseases, such as psoriasis, rheumatoid arthritis, systemic lupus erythematosus (SLE), ankylosing spondylitis, ulcerative colitis, Crohn's disease, type 1 diabetes, and multiple sclerosis, can be obtained in humans with an inactivated Tyk2 variant, and inhibition of Tyk2 is also expected to have an effect on cancer and COVID-19 infection (Non Patent Literatures 3 and 4).

However, the use of JAK inhibitors that non-specifically inhibit tyrosine kinases that constitute the JAK family increases the risk of side effects such as the suppression of the hematopoietic system, and therefore JAK inhibitors that selectively inhibit one of the JAKs have been developed (Non Patent Literatures 1 and 5). Such JAK inhibitors are quickly excreted from the tissues, since they are low molecular weight compounds, and thus require high doses in order to increase local concentrations, which may easily cause side effects.

A degradation inducer, i.e., a degrader, is a technology that uses the intracellular ubiquitin-proteasome system to degrade target proteins. This technology has received attention in recent years, because it can target for degradation of such a protein that has no active center and also can be applied to such a protein that has mutation in its active center and thus cannot be suppressed by existing inhibitors (Patent Literature 1 and Non Patent Literature 6). In addition, since degraders have larger molecular weight as compared with inhibitors, which are low molecular weight compounds, the local retention time of degraders is longer and the local concentration of degraders can be easily maintained, which may lower doses and reduce side effects.

### Citation List

### Patent Literature

Patent Literature 1: JP WO2020/009176

### Non Patent Literature

Non Patent Literature 1: Drug Delivery System, 35-5, 376-383, 2020
Non Patent Literature 2: Nature review drug discovery, 2017, 16, 843
Non Patent Literature 3: Immuno Horizons, 2021, 5(2), 70-80
Non Patent Literature 4: Nature, 591, 92-98, 2021
Non Patent Literature 5: J. Med Chem, 2019, 62, 8973-8995
Non Patent Literature 6: YAKUGAKU ZASSHI, 138, 1135-1143 (2018)

### Summary of Invention

An object of the present invention is to provide a novel compound that induces selective degradation of a non-receptor tyrosine kinase (and especially a tyrosine kinase that constitute the JAK family), or a pharmaceutically acceptable salt thereof, and another object is to provide a degrader of a non-receptor tyrosine kinase or medicament containing such a compound or a pharmaceutically acceptable salt thereof. Still another object of the present invention is to provide a method of improving, preventing and/or treating a condition or disease requiring modulation, such as the suppression, of activation of cytokine signaling pathways, the method including administering such a degrader or medicament. Yet another object of the present invention is to provide a method for screening substances having the degradation inducing activity toward non-receptor tyrosine kinases.

As a result of research, the present inventors have discovered that a compound in which a ligand of VHL (von Hippel-Lindau), which is the substrate recognition subunit of an E3 ubiquitin ligase, is bound to a pyridyl or pyridazinyl compound via a linker can induce selective degradation of tyrosine kinases that are part of the JAK family, and thereby achieved the present invention.

Specifically, the summary of the present invention is as follows.
[1] A compound represented by formula I: 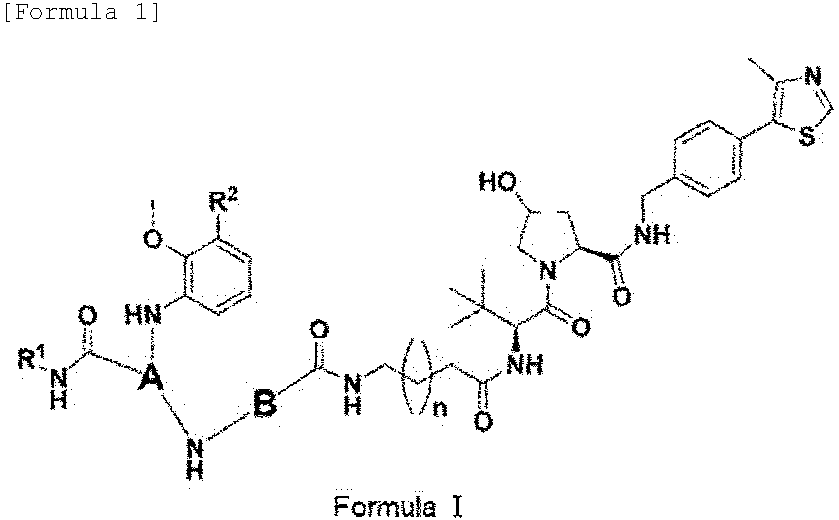 wherein R¹ is a hydrogen atom or a C₁₋₃ alkyl group optionally substituted with one or more deuterium atoms; R² is CONHR³ (wherein R³ is a C₁₋₃ alkyl group optionally substituted with one or more OH groups) or a triazole group optionally substituted with one or more C₁₋₃ alkyl groups; A is a pyridyl group or a pyridazinyl group; B is 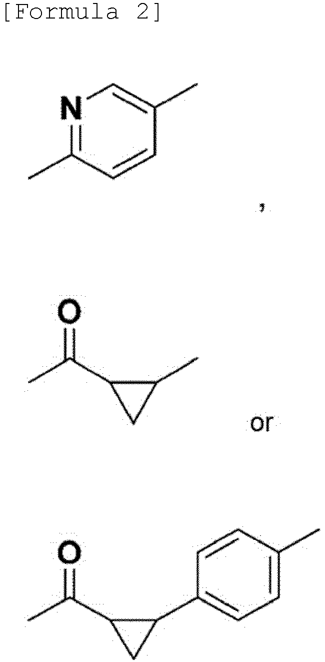 and n is an integer between 0 and 12, or a pharmaceutically acceptable salt thereof.
[2] The compound according to item [1] or a pharmaceutically acceptable salt thereof, wherein in formula I, R¹ is a hydrogen atom, a methyl group, or a methyl group substituted with 3 deuterium atoms, and R² is CONHCH₃, CONHCH₂OH, CONHC₂H₅, CONHC₂H₄OH, or a triazole group substituted with one methyl group.
[3] The compound according to [1] represented by formula II: 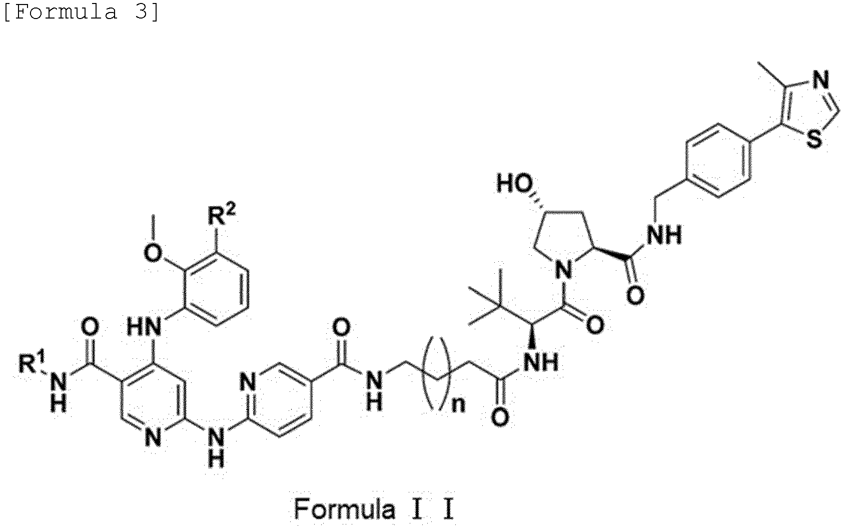 wherein R¹ is a hydrogen atom, a methyl group, or an ethyl group; R² is CONHR³ (wherein R³ is as defined in [1]) or a triazole group optionally substituted with one or more methyl groups or ethyl groups; and n is an integer between 1 and 12, or a pharmaceutically acceptable salt thereof.
[4] The compound according to [1] represented by formula III: 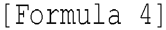 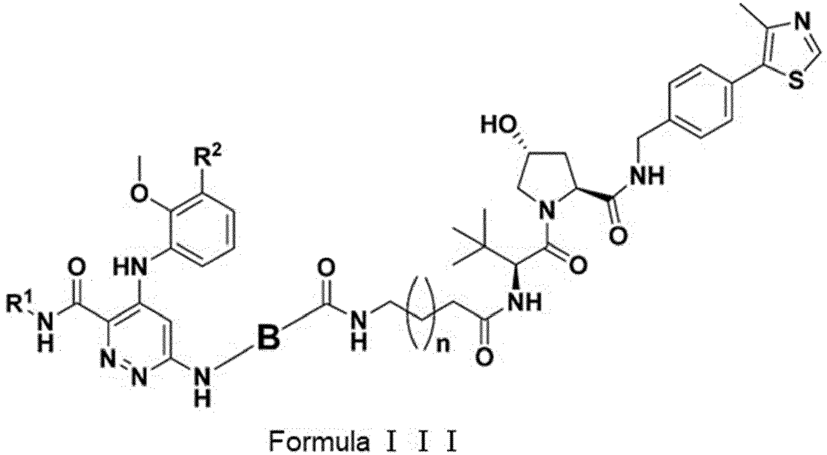 wherein R¹ is a methyl group substituted with three deuterium atoms; R² is a triazole group optionally substituted with one or more methyl or ethyl groups; B is 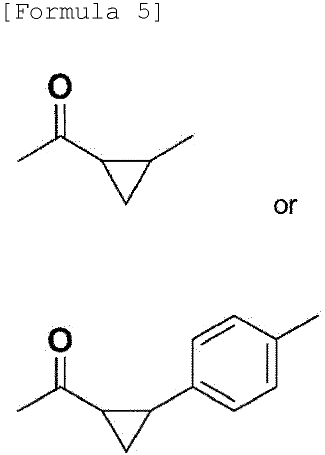 and n is an integer between 1 and 12, or a pharmaceutically acceptable salt thereof.
[5] A degrader of a non-receptor tyrosine kinase, comprising the compound according to any one of [1] to [4] or a pharmaceutically acceptable salt thereof.
[6] The degrader according to [5], wherein the degrader selectively degrades at least one non-receptor tyrosine kinase selected from the group consisting of JAK1, JAK2, JAK3, and Tyk2.
[7] A medicament comprising the compound according to any one of [1] to [4] or a pharmaceutically acceptable salt thereof.
[8] The medicament according to [7], wherein the medicament is used for improving, preventing and/or treating a condition or disease requiring the suppression of activation of the signaling pathway of at least one cytokine selected from the group consisting of IFNα, IFNβ, IFNγ, IL-2, IL-4, IL-6, IL-7, IL-9, IL-10, IL-11, IL-12, IL-15, IL-19, IL-20, IL-21, IL-22, IL-23, IL-27, G-CSF, LIF, and oncostatin M.
[9] The medicament according to [7] or [8], wherein the medicament is a therapeutic agent for preventing or treating an autoimmune disease, an inflammatory disease, COVID-19 infection, cancer, atopic dermatitis, or alopecia.
[10] A method of improving, preventing and/or treating a condition or disease requiring the suppression of activation of the signaling pathway of at least one cytokine selected from the group consisting of IFNα, IFNβ, IFNγ, IL-2, IL-4, IL-6, IL-7, IL-9, IL-10, IL-11, IL-12, IL-15, IL-19, IL-20, IL-21, IL-22, IL-23, IL-27, G-CSF, LIF, and oncostatin M, the method comprising administering the medicament according to [7] to a subject in need thereof.
[11] A use of the compound according to any one of [1] to [4] or a pharmaceutically acceptable salt thereof, for producing the medicament according to any one of [7] to [9] .
[12] A method of screening a substance inducing the degradation of a non-receptor tyrosine kinase, comprising the following steps: (1) a step of detecting the degradation-inducing activity of a candidate substance toward a non-receptor tyrosine kinase by coexisting the candidate substance with the non-receptor tyrosine kinase and; and (2) a step of selecting the candidate substance that degraded the non-receptor tyrosine kinase in step (1) as a degradation-inducing substance; provided that the candidate substance is not selected as a degradation-inducing substance if the degradation activity of the candidate substance is less than the degradation activity of the compound of the following formula IV.

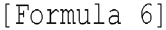

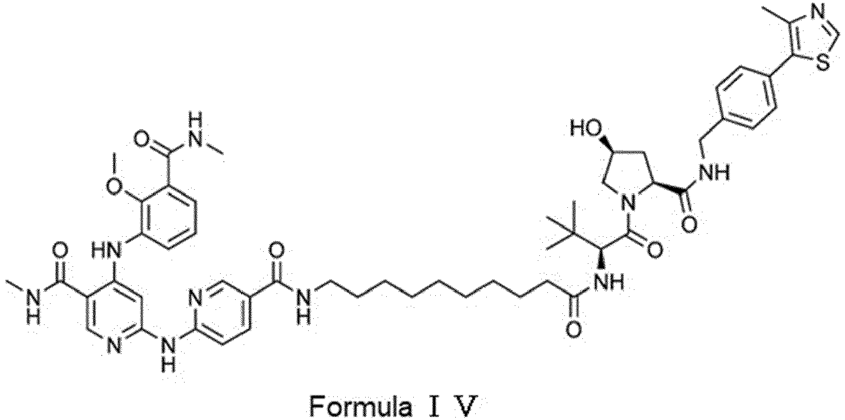

Compounds represented by the above formula I or the pharmacologically acceptable salt thereof provided by the present invention have the degradation-inducing activity selectively toward non-receptor tyrosine kinases, especially JAKs, and are thus capable of modulation, such as the suppression, of activation of cytokine signaling pathways for which JAKs are responsible, and accordingly, they are useful as medicaments for the prevention or treatment of, for example, autoimmune diseases, inflammatory diseases, COVID-19 infection, cancer, atopic dermatitis, and alopecia, as well as useful as reagents for research purposes.

### Brief Description of Drawings

[Figure 1] Figure 1 shows the results of the protein levels of JAK1, JAK2, JAK3, Tyk2, and GAPDH in Jurkat cells after 6 hours of treatment with each test compound, as detected by chemiluminescent Western blotting.
[Figure 2] Figure 2 is a graph created by quantifying the detection results of the chemiluminescent Western blotting shown in Figure 1.

### Description of Embodiments

As used herein, the present invention will be further described in detail using preferred embodiments, but the present invention is not limited thereto in any way.

In one aspect, the present invention relates to a compound represented by the following formula I or a pharmaceutically acceptable salt thereof.

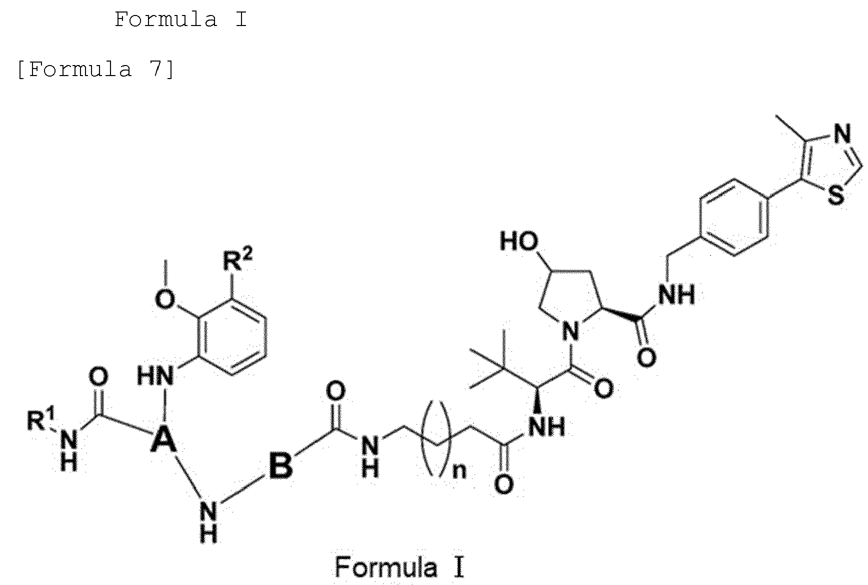

R¹ is a hydrogen atom or a C₁₋₃ alkyl group optionally substituted with one or more deuterium atoms. R¹ is preferably a hydrogen atom or a methyl or ethyl group optionally substituted with three deuterium atoms, more preferably a hydrogen atom, a methyl group, or a methyl or ethyl group substituted with three deuterium atoms, and even more preferably a hydrogen atom, a methyl group, or a methyl group substituted with three deuterium atoms.

R² is CONHR³ (wherein R³ is a C₁₋₃ alkyl group optionally substituted with one or more OH groups) or a triazole group optionally substituted with one or more C₁₋₃ alkyl groups. R² is preferably CONHCH₃, CONHCH₂OH, CONHC₂H₅, CONHC₂H₄OH, or a triazole group optionally substituted with one or more methyl or ethyl groups, and more preferably CONHCH₃, CONHC₂H₄OH, or a triazole group optionally substituted with one methyl group.

A is a pyridyl group or a pyridazinyl group. When R¹ is a hydrogen atom or a C₁₋₃ alkyl group, A is preferably a pyridyl group. When R¹ is a C₁₋₃ alkyl group substituted with one or more deuterium atoms, A is preferably a pyridazinyl group.

B is the following: 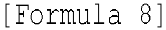 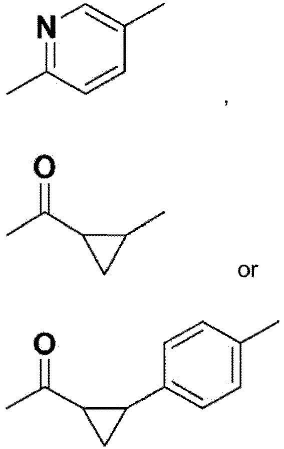 When R¹ is a hydrogen atom or a C₁₋₃ alkyl group, B is preferably the following: 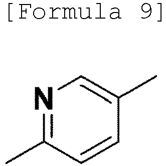 When R¹ is a C₁₋₃ alkyl group substituted with one or more deuterium atoms, B is preferably the following:

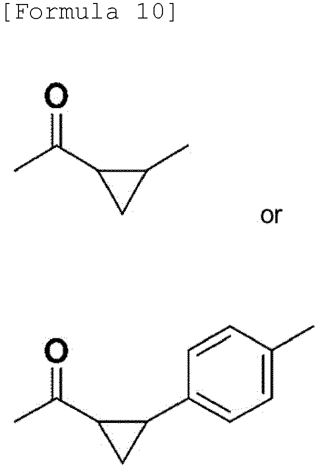

In formula I, n is an integer between 0 and 12. Preferably, n is 1 or more, and may be 2 or more, 3 or more, or 4 or more, but is preferably 11 or less, and may be 10 or less, 9 or less, or 8 or less. For example, n may be in the range of 0 to 12, 1 to 12, 2 to 12, 3 to 12, 4 to 12, 5 to 12, 6 to 12, 0 to 11, 1 to 11, 2 to 11, 3 to 11, 4 to 11, 5 to 11, 6 to 11, 0 to 10, 1 to 10, 2 to 10, 3 to 10, 4 to 10, 5 to 10, 6 to 10, 0 to 9, 1 to 9, 2 to 9, 3 to 9, 4 to 9, 5 to 9, 6 to 9, 0 to 8, 1 to 8, 2 to 8, 3 to 8, 4 to 8, 5 to 8, or 6 to 8. Preferably, n is in the range of 3 to 9, 4 to 9, 5 to 9, 6 to 9, 3 to 8, 4 to 8, 5 to 8, or 6 to 8, and more preferably in the range of 5 to 8, 6 to 9, or 6 to 8.

In formula I, the stereo configuration of the two chiral carbons on the pyrrolidyl group may be 2S,4R or 2S,4S. In one embodiment of the present invention, the stereo configuration of the two chiral carbons on the pyrrolidyl group is preferably 2S,4R. In another embodiment of the present invention, the stereo configuration of the two chiral carbons on the pyrrolidyl group is preferably 2S,4S.

As used herein, "C₁₋₃ alkyl group" refers to linear and branched alkyl groups having 1 to 3 carbon atoms (e.g., methyl, ethyl, propyl, and isopropyl groups).

As used herein, the "pharmaceutically acceptable salt" is not particularly limited as long as it is acceptable as a medicament, and can be appropriately selected according to the purpose. Examples thereof include salts with inorganic acids such as hydrochloric acid and sulfuric acid, salts with organic acids such as maleic acid and tartaric acid, salts with alkali metals such as sodium and potassium, salts with alkaline earth metals such as calcium and magnesium, and salts with organic bases such as diethylamine and diethanolamine. In the case that the compound of the present invention becomes a hydrate, for example, by being left in the atmosphere, such a hydrate is also encompassed by the salt of the present invention.

In one embodiment, the compound represented by formula I provided by the present invention or a pharmaceutically acceptable salt of the compound is a compound represented by formula II: 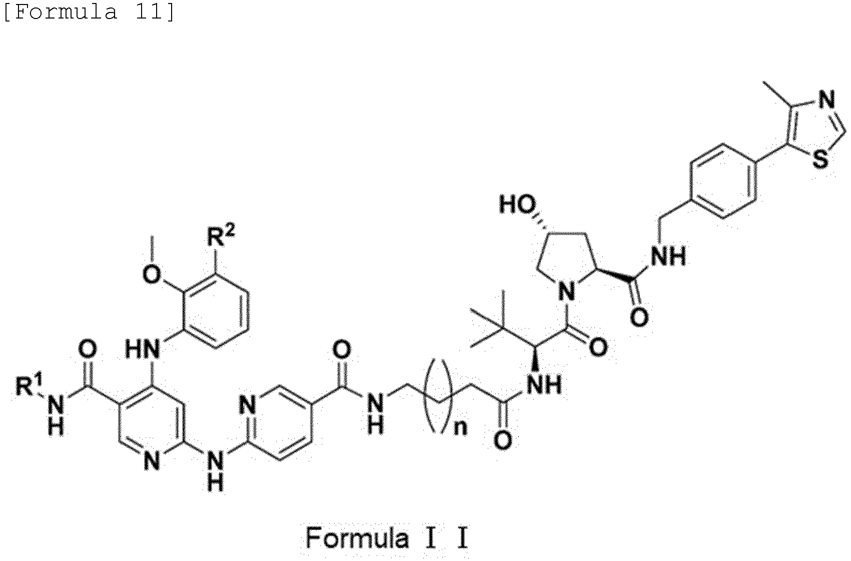 wherein R¹ is a hydrogen atom, a methyl group, or an ethyl group; R² is CONHR³ (wherein R³ is a C₁₋₃ alkyl group optionally substituted with one or more OH groups) or a triazole group optionally substituted with one or more methyl or ethyl groups; and n is an integer between 1 and 12, or a pharmaceutically acceptable salt thereof. In formula II, R¹ is preferably a hydrogen atom or a methyl group, R² is preferably CONHR³ (wherein R³ is a methyl or ethyl group optionally substituted with one or more OH groups) or a triazole group optionally substituted with one or more methyl or ethyl groups, and n is preferably in the range of 1 to 12, 2 to 12, 3 to 12, 4 to 12, 5 to 12, 6 to 12, 1 to 11, 2 to 11, 3 to 11, 4 to 11, 5 to 11, 6 to 11, 1 to 10, 2 to 10, 3 to 10, 4 to 10, 5 to 10, 6 to 10, 1 to 9, 2 to 9, 3 to 9, 4 to 9, 5 to 9, 6 to 9, 1 to 8, 2 to 8, 3 to 8, 4 to 8, 5 to 8, or 6 to 8, more preferably in the range of 3 to 9, 4 to 9, 5 to 9, 6 to 9, 3 to 8, 4 to 8, 5 to 8, or 6 to 8, and even more preferably in the range of 5 to 8, 6 to 9, or 6 to 8.

In one embodiment, the compound represented by formula I or a pharmaceutically acceptable salt thereof is a compound represented by formula III: 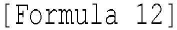 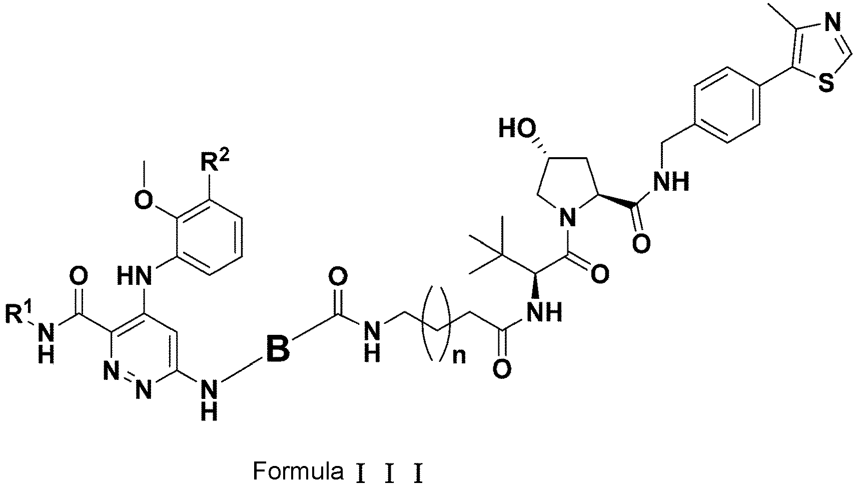 wherein R¹ is a methyl group substituted with three deuterium atoms; R² is a triazole group optionally substituted with one or more methyl or ethyl groups; B is 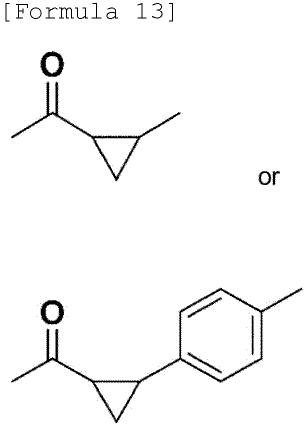 and n is an integer between 1 and 12, or a pharmaceutically acceptable salt thereof. In formula III, R² is preferably a triazole group optionally substituted with one methyl or ethyl group, and more preferably a triazole group substituted with one methyl or ethyl group, and n is preferably in the range of 1 to 12, 2 to 12, 3 to 12, 4 to 12, 5 to 12, 6 to 12, 1 to 11, 2 to 11, 3 to 11, 4 to 11, 5 to 11, 6 to 11, 1 to 10, 2 to 10, 3 to 10, 4 to 10, 5 to 10, 6 to 10, 1 to 9, 2 to 9, 3 to 9, 4 to 9, 5 to 9, 6 to 9, 1 to 8, 2 to 8, 3 to 8, 4 to 8, 5 to 8, or 6 to 8, more preferably in the range of 3 to 9, 4 to 9, 5 to 9, 6 to 9, 3 to 8, 4 to 8, 5 to 8, or 6 to 8, and even more preferably in the range of 5 to 8, 6 to 9, or 6 to 8.

As used herein, the compounds represented by formula I, II, and III and the pharmaceutically acceptable salts thereof are sometimes collectively referred to as the "compound of the present invention."

In one aspect of the present invention, a degrader of non-receptor tyrosine kinase comprising the compound of the present invention is provided. It is preferred that the degrader of non-receptor tyrosine kinase comprising the compound of the present invention selectively degrades at least one non-receptor tyrosine kinase selected from the group consisting of JAK1, JAK2, JAK3, and Tyk2. In one embodiment, the degrader of non-receptor tyrosine kinase comprising the compound of the present invention is a selective degrader of JAK1 and Tyk2. In one embodiment, the degrader of non-receptor tyrosine kinase comprising the compound of the present invention is a selective degrader of Tyk2.

In one aspect of the present invention, a medicament comprising the compound of the present invention is provided. The medicament of the present invention can be used for improving, preventing and/or treating conditions or diseases that require modulation, such as the suppression, of the activation of cytokine signaling pathways, since the compound of the present invention is capable of modulating, such as suppressing, the activation of cytokine signaling pathways mediated by a non-receptor tyrosine kinase by degrading the non-receptor tyrosine kinase. Since JAK1 and Tyk2 mediate the signaling pathways of, for example, IFNα,IFNβ, IFNγ, IL-6, IL-10, IL-11, IL-12, IL-19, IL-20, IL-22, IL-23, LIF, and oncostatin M, the medicament of the present invention is believed to help prevent or treat autoimmune diseases, inflammatory diseases, COVID-19 infection, cancer, atopic dermatitis, alopecia, and others in which these cytokines are involved. More specific examples include autoimmune diseases such as psoriasis, rheumatoid arthritis, systemic lupus erythematosus, ankylosing spondylitis, ulcerative colitis, Crohn's disease, type 1 diabetes, and multiple sclerosis, and cancer such as T-cell acute lymphoblastic leukemia, anaplastic large cell lymphoma, and malignant nerve sheath tumor. As used herein, "cancer" refers to all malignant neoplasms, regardless of whether epithelial or non-epithelial.

The medicament of the present invention can be administered to any animals, but preferably the subject of administration is a mammal, and more preferably a human.

As the medicament of the present invention, the compound of the present invention may be administered as it is, or it may be administered after being formulated by a conventional method in combination with a formulation carrier conventionally used by those skilled in the art.

As the formulation carrier, for example, an excipient, a binder, a bulking agent, a disintegrant, a surfactant, a lubricant, a dispersant, a buffer, a preservative, a flavoring agent, a fragrance, a coating agent, a diluent, or a coloring agent can be appropriately selected according to the form of the medicament and the purpose, such as the purpose of imparting sustained release properties to the medicament.

The dosage form of the medicament of the present invention is not particularly limited and can be appropriately selected as needed. Examples thereof include, but are not limited to, oral formulations such as tablets, capsules, granules, powders, sustained-release formulations, oral liquids, suspensions, syrups, oral tablets, and oral sprays; and parenteral formulations such as injections, ointments, inhalants, ophthalmic solutions, ophthalmic ointments, nasal drops, suppositories, external solid preparations, external liquid preparations, sprays, creams, gels, and patches.

The administration route of the medicament of the present invention is appropriately selected, taking into consideration the type and extent of the condition or disease to be improved, prevented and/or treated, and the age, gender, drug sensitivity, and the like of the subject. Examples of the administration route include oral, intravenous, transnasal, transdermal, transmucosal, intra-articular, transpulmonary, transbronchial, transrectal, and ocular administration.

In one aspect, the compound of the present invention can also be used as a reagent for research purposes. In one embodiment, the compound of the present invention can be used as a positive control for screening substances having the degradation-inducing activity toward non-receptor tyrosine kinases. In another embodiment, the compound of the present invention can be used as a negative control for screening substances having the degradation-inducing activity toward non-receptor tyrosine kinases.

The compound of the present invention can be produced using appropriate intermediates, and known compounds and reagents, for example by the method shown in the following scheme. In the following scheme, Compound (11) corresponds to the compound represented by formula I, where R¹, R², A and B are the same as defined above for formula I.

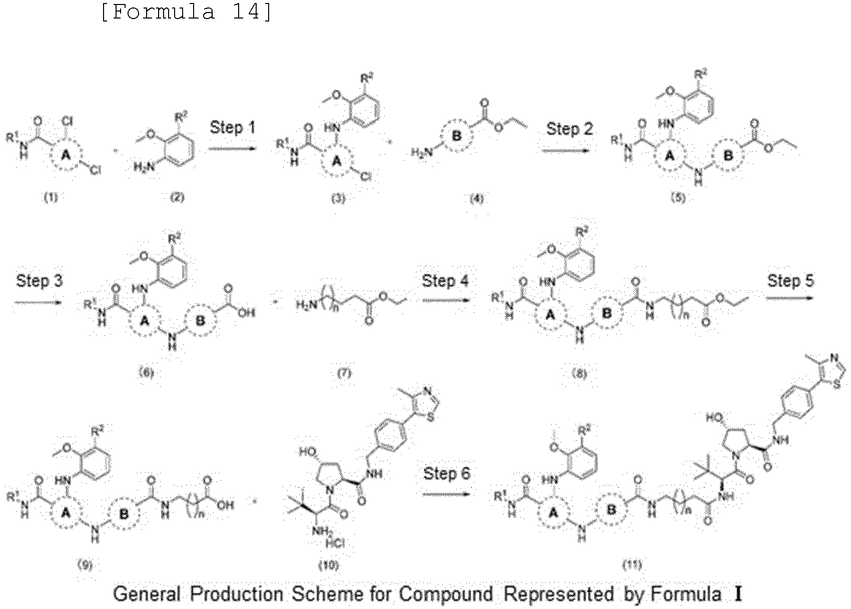

### (Method)

### Step 1

Compound (3) can be obtained by an aromatic nucleophilic substitution reaction between Compound (1) and Compound (2) according to a known method (J. Med Chem 2019, 62, 8973-8995).

### Step 2

Compound (5) can be obtained by a Buchwald-Hartwig coupling reaction between Compound (3) and Compound (4).

### Step 3

Compound (6) can be obtained by subjecting Compound (5) to an alkaline hydrolysis reaction.

### Step 4

Compound (8) can be obtained by an amidation reaction between Compound (6) and a commercially available reagent (7).

### Step 5

Compound (9) can be obtained by subjecting Compound (8) to an alkaline hydrolysis reaction.

### Step 6

Target Compound (11) can be obtained by subjecting Compound (9) to an amidation reaction with Compound (10) obtained according to a known method (WO2016/118666).

### Examples

Hereinafter, the present invention will be described in detail by way of Reference Examples, Examples, Comparative Examples, and a Test Example, but the present invention is not limited thereto. In the following Reference Examples, Examples, Comparative Examples, and Test Example, commercially available reagents and equipment were used unless otherwise noted.

The synthesis scheme of Examples 1 to 7 is shown below.

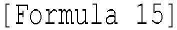

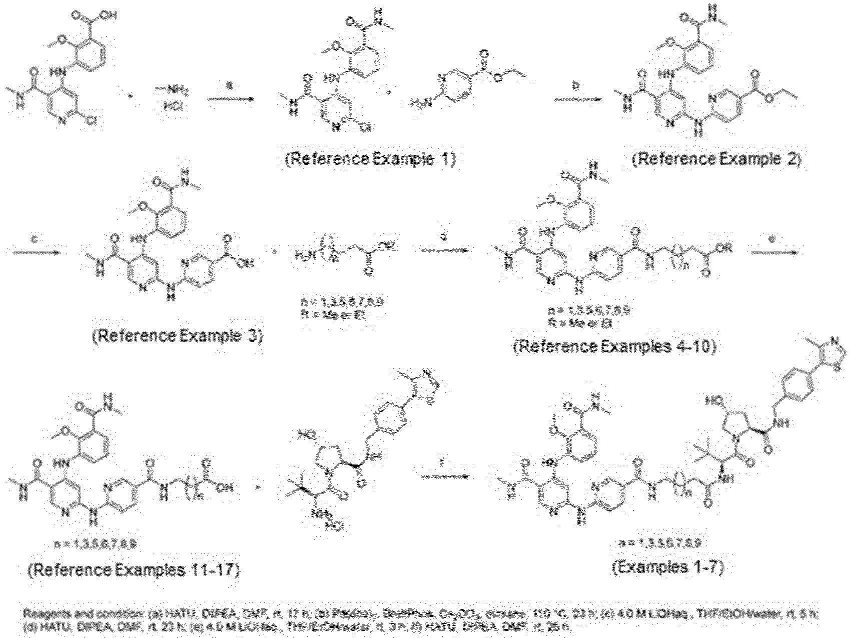

### Synthesis Scheme of Examples 1 to 7

### Reference Example 1: 6-Chloro-4-((2-methoxy-3-(methylcarbamoyl)phenyl)amino)-N-methylnicotinamide

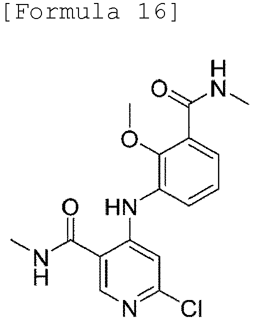

To a 3-((2-chloro-5-(methylcarbamoyl)pyridyl-4-yl)amino)-2-methoxybenzoic acid (synthesized according to the method described in J. Med Chem. 2019, 62, 8973.) (2.59 g, 7.71 mmol) solution in N,N-dimethylformamide (DMF) (40 mL) were added methylamine hydrochloride (0.78 g, 11.6 mmol) and N,N-diisopropylethylamine (DIPEA) (2.99 g, 23.1 mmol), followed by cooling in an ice bath. O-(7-Azabenzotriazol-1-yl)-N,N,N',N'-tetramethyluronium hexafluorophosphate (HATU) (4.40 g, 11.6 mmol) was then added to the solution at the same temperature, followed by stirring for 10 minutes. The reaction solution was heated to room temperature and stirred for 17 hours. A saturated sodium bicarbonate solution and a solvent mixture of ethyl acetate/methanol were added to the reaction solution, followed by stirring for 30 minutes. From the resultant, the organic layer was separated, washed with water twice and dried over magnesium sulfate, followed by removal of the solvent under reduced pressure. The thus-obtained residue was purified by silica gel column chromatography (methanol/chloroform = 15%) and the solvent was distilled off under reduced pressure. To the thus-obtained solid, tert-Butyl methyl ether was added, followed by filtration to give the title compound (2.61 g, 7.48 mmol, 97% yield).
¹H-NMR (d₆-DMSO, 400 MHz) δ 10.6 (s, 1H), 8.83 (d, J = 4.4 Hz, 1H), 8.51 (s, 1H), 8.26 (dd, J = 8.8 Hz, 4.4 Hz, 1H), 7.54 (dd, J = 7.6 Hz, 2.0 Hz, 1H), 7.35 (dd, J = 7.6 Hz, 1.6 Hz, 1H), 7.23 (t, J = 7.6 Hz, 1H), 6.86 (s, 1H), 3.69 (s, 3H), 2.81 (d, J = 4.8 Hz, 3H), 2.79 (d, J = 4.4 Hz, 3H); MS (EI) m/z = 348 (M⁺).

### Reference Example 2: Ethyl-6-((4-(2-methoxy-3-(methylcarbamoyl)anilino)-5-(methylcarbamoyl)-2-pyridyl)amino)pyridine-3-carboxylate

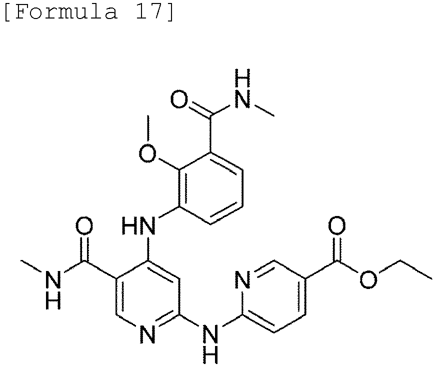

To a 6-chloro-4-((2-methoxy-3-(methylcarbamoyl)phenyl)amino)-N-methylnicotinamide (Reference Example 1) (1.80 g, 5.16 mmol) solution in 1,4-dioxane (30 mL) were sequentially added ethyl 6-aminopyridine-3-carboxylate (1.29 g, 7.74 mmol), bis(benzylideneacetone)palladium(0) (Pd(dba)₂) (0.45 g, 0.77 mmol), 2-(dicyclohexylphosphino)-3,6-dimethoxy-2',4',6'-triisopropyl-1,1'-biphenyl (BrettPhos) (0.42 g, 0.77 mmol) and cesium carbonate (Cs₂CO₃) (3.36 g, 10.3 mmol), followed by stirring at 110°C for 23 hours under a nitrogen atmosphere. The reaction solution was allowed to cool to room temperature, and mixed with water, followed by extraction twice with ethyl acetate. The combined extracts were washed with water, followed by removal of the solvent under reduced pressure. The resulting residue was purified by silica gel column chromatography (methanol/chloroform = 10%) and the solvent was distilled off under reduced pressure to give the title compound (1.07 g, 2.24 mmol, 43% yield). ¹H-NMR (d₆-DMSO, 400 MHz) δ 10.7 (s, 1H), 10.3 (s, 1H), 8.70 (d, J = 2.0 Hz, 1H), 8.59 (dd, J = 8.8 Hz, 4.4 Hz, 1H), 8.53 (s, 1H), 8.26 (dd, J = 9.2 Hz, 4.4 Hz, 1H), 8.12 (dd, J = 8.8 Hz, 2.4 Hz, 1H), 7.84 (s, 1H), 7.75 (d, J = 8.8 Hz, 1H), 7.68-7.63 (m, 1H), 7.29-7.24 (m, 2H), 4.30 (q, J =7.2 Hz, 2H), 3.73 (s, 3H), 2.81 (d, J =4.4 Hz, 3H), 2.80 (d, J =4.4 Hz, 3H), 1.32 (d, J =7.2 Hz, 3H); MS (EI) m/z = 478 (M⁺).

### Reference Example 3: 6-((4-(2-Methoxy-3-(methylcarbamoyl)anilino)-5-(methylcarbamoyl)-2-pyridyl)amino)pyridine-3-carboxylic acid

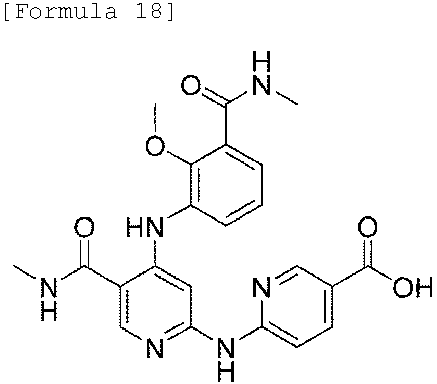

Ethyl-6-((4-(2-methoxy-3-(methylcarbamoyl)anilino)-5-(methylcarbamoyl)-2-pyridyl)amino)pyridine-3-carboxylate (Reference Example 2) (1.07 g, 2.24 mmol) was dissolved in a solvent mixture of ethanol (12 mL), tetrahydrofuran (THF) (12 mL) and purified water (12 mL), followed by the addition of 4.0 mol/L lithium hydroxide solution (LiOH) (1.96 mL, 7.83 mmol) and stirring for 5 hours at room temperature. Water was added to the reaction solution, which was then neutralized with 6.0 mol/L hydrochloric acid, and the precipitated solid was collected by filtration. The thus-obtained solid was dried under reduced pressure at 80°C for 2 hours to give the title compound (0.95 g, 2.10 mmol, 94% yield).
¹H-NMR (d₆-DMSO, 400 MHz) δ 12.8 (brs, 1H), 10.7 (s, 1H), 10.2 (s, 1H), 8.68 (d, J = 2.4 Hz, 1H), 8.58 (dd, J = 8.8 Hz, 4.4 Hz, 1H), 8.53 (s, 1H), 8.26 (dd, J = 9.2 Hz, 4.4 Hz, 1H), 8.10 (dd, J = 8.8 Hz, 2.4 Hz, 1H), 7.92 (s, 1H), 7.69-7.64 (m, 2H), 7.29-7.23 (m, 2H), 3.73 (s, 3H), 2.80 (d, J =4.4 Hz, 3H), 2.80 (d, J =4.4 Hz, 3H); MS (EI) m/z = 450 [M⁺].

### Reference Example 4: Ethyl 4-(6-((4-(2-methoxy-3-(methylcarbamoyl)phenyl)amino)-5-(methylcarbamoyl)pyridin-2-yl)amino)nicotinamide)butanoate

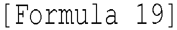

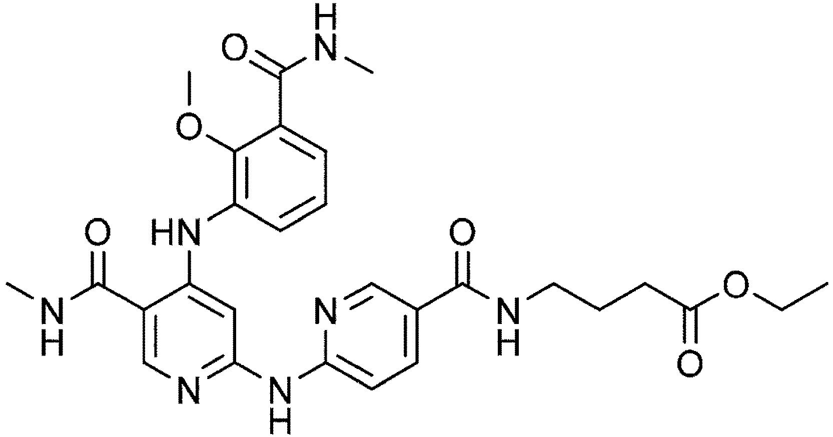

To a 6-((4-(2-methoxy-3-(methylcarbamoyl)anilino)-5-(methylcarbamoyl)-2-pyridyl)amino)pyridine-3-carboxylic acid (Reference Example 3) (100 mg, 0.22 mmol) solution in N,N-dimethylformamide (DMF) (2.0 mL) were added ethyl 4-aminobutyrate hydrochloride (74.4 mg, 0.44 mmol) and N,N-diisopropylethylamine (DIPEA) (172 mg, 1.33 mmol), followed by cooling in an ice bath. Then, O-(7-azabenzotriazol-1-yl)-N,N,N',N'-tetramethyluronium hexafluorophosphate (HATU) (127 mg, 0.33 mmol) was added to the solution at the same temperature, followed by stirring for 10 minutes. The reaction solution was heated to room temperature, stirred for 23 hours, mixed with a saturated sodium bicarbonate solution and a solvent mixture of ethyl acetate/methanol, and stirred for 30 minutes, followed by separation of the organic layer. The resultant aqueous layer was extracted twice with a solvent mixture of ethyl acetate/methanol, the combined organic layers were washed three times with water, and the organic layer was dried over magnesium sulfate, followed by removal of the solvent under reduced pressure. The thus-obtained residue was purified by silica gel column chromatography (methanol/chloroform = 15%) and the solvent was distilled off under reduced pressure. Diethyl ether was added to the thus-obtained solid, which was then collected by filtration to give the title compound (89.0 mg, 0.16 mmol, 71% yield).
¹H-NMR (d₄-MeOH 400 MHz) δ 8.63 (dd, J = 2.4 Hz, 0.8 Hz, 1H), 8.40 (s, 1H), 8.03 (dd, J = 8.8 Hz, 2.4 Hz, 1H), 7.91 (s, 1H), 7.75 (dd, J = 8.4 Hz, 2.0 Hz, 1H), 7.50 (dd, J = 8.0 Hz, 1.6 Hz, 1H), 7.45 (d, J = 8.8 Hz, 1H), 7.32 (t, J = 8.4 Hz, 1H), 4.11 (q, J = 7.2 Hz, 2H), 3.81 (s, 3H), 3.41 (t, J = 6.8 Hz, 2H), 2.97 (s, 3H), 2.92 (s, 3H), 2.41 (t, J = 7.2 Hz, 2H), 1.95-1.88 (m, 2H), 1.23 (t, J = 7.2 Hz, 3H); MS (EI) m/z = 563 (M⁺).

### Reference Example 5: Ethyl 6-(6-((4-(2-methoxy-3-(methylcarbamoyl)phenyl)amino)-5-(methylcarbamoyl)pyridin-2-yl)amino)nicotinamide)hexanoate

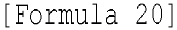

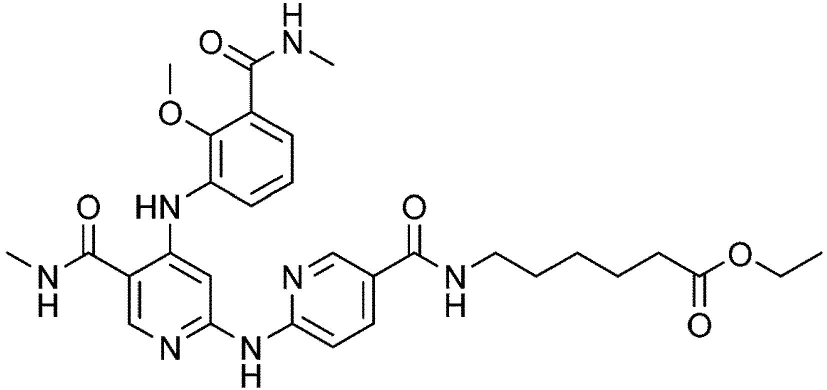

Using 6-((4-(2-methoxy-3-(methylcarbamoyl)anilino)-5-(methylcarbamoyl)-2-pyridyl)amino)pyridine-3-carboxylic acid (Reference Example 3) (100 mg, 0.22 mmol) and ethyl 6-aminohexanoate hydrochloride (86.9 mg, 0.44 mmol), the same procedure as in Reference Example 4 was conducted to give the title compound (119 mg, 0.20 mmol, 91% yield).
¹H-NMR (d₄-MeOH 400 MHz) δ 8.64 (d, J = 1.6 Hz, 1H), 8.40 (s, 1H), 8.04 (dd, J = 8.8 Hz, 2.4 Hz, 1H), 7.90 (s, 1H), 7.74 (dd, J = 8.0 Hz, 1.6 Hz, 1H), 7.51 (dd, J = 8.0 Hz, 1.6 Hz, 1H), 7.42 (d, J = 8.8 Hz, 1H), 7.32 (t, J = 8.0 Hz, 1H), 4.14-4.07 (m, 2H), 3.81 (s, 3H), 3.37 (t, J = 6.8 Hz, 2H), 2.97 (s, 3H), 2.93 (s, 3H), 2.36-2.32 (m, 2H), 1.70-1.60 (m, 4H), 1.45-1.37 (m, 2H), 1.25-1.21 (m, 3H); MS (EI) m/z = 591 (M⁺).

### Reference Example 6: Ethyl 8-(6-((4-(2-methoxy-3-(methylcarbamoyl)phenyl)amino)-5-(methylcarbamoyl)pyridin-2-yl)amino)nicotinamide)octanoate

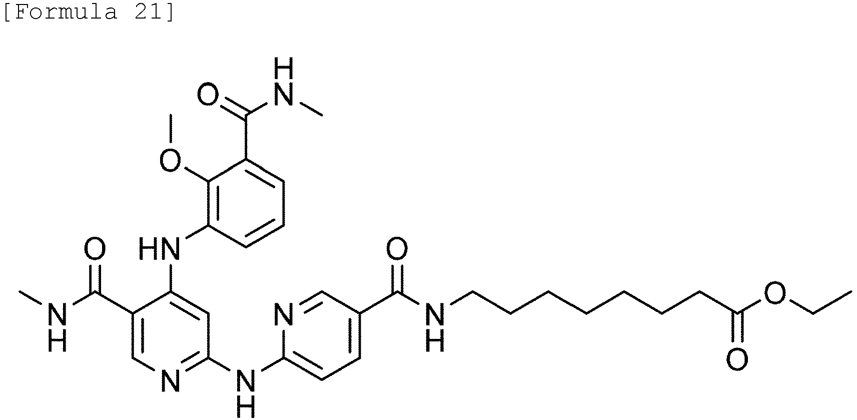

Using 6-((4-(2-methoxy-3-(methylcarbamoyl)anilino)-5-(methylcarbamoyl)-2-pyridyl)amino)pyridine-3-carboxylic acid (Reference Example 3) (100 mg, 0.22 mmol) and ethyl 8-aminooctanoate hydrochloride (99.3 mg, 0.44 mmol), the same procedure as in Reference Example 4 was performed to give the title compound (146 mg, 0.24 mmol, 106% yield).
¹H-NMR (d₄-MeOH 400 MHz) δ 8.66 (d, J = 2.0 Hz, 1H), 8.40 (s, 1H), 8.06 (dd, J = 8.8 Hz, 2.4 Hz, 1H), 7.72 (dd, J = 8.0 Hz, 1.6 Hz, 1H), 7.67 (brs, 1H), 7.53 (dd, J = 8.0 Hz, 1.6 Hz, 1H), 7.37 (brd, J = 8.8 Hz, 1H), 7.32 (t, J = 8.0 Hz, 1H), 4.13-4.07 (m, 2H), 3.81 (s, 3H), 3.36 (t, J = 7.2 Hz, 2H), 2.97 (s, 3H), 2.93 (s, 3H), 2.33-2.28 (m, 2H), 1.63-1.58 (m, 4H), 1.41-1.32 (m, 6H), 1.25-1.21 (m, 3H); MS (EI) m/z = 619 (M⁺).

### Reference Example 7: Ethyl 9-(6-((4-(2-methoxy-3-(methylcarbamoyl)phenyl)amino)-5-(methylcarbamoyl)pyridin-2-yl)amino)nicotinamide)nonanoate

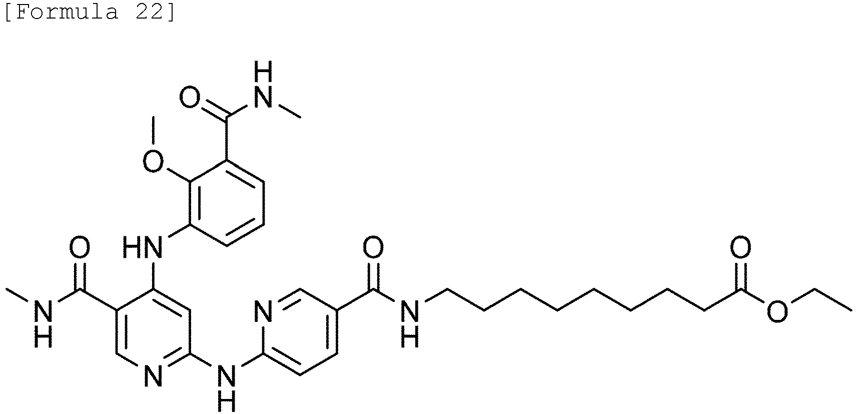

Using 6-((4-(2-methoxy-3-(methylcarbamoyl)anilino)-5-(methylcarbamoyl)-2-pyridyl)amino)pyridine-3-carboxylic acid (Reference Example 3) (100 mg, 0.22 mmol) and ethyl 9-aminononanoate hydrochloride (106 mg, 0.44 mmol), the same procedure as in Reference Example 4 was performed to give the title compound (117 mg, 0.18 mmol, 83% yield).
¹H-NMR (d₄-MeOH 400 MHz) δ 8.63 (d, J = 1.6 Hz, 1H), 8.40 (s, 1H), 8.03 (dd, J = 8.8 Hz, 2.8 Hz, 1H), 7.87 (brs, 1H), 7.74 (dd, J = 8.4 Hz, 1.6 Hz, 1H), 7.50 (dd, J = 8.0 Hz, 1.6 Hz, 1H), 7.43 (d, J = 8.8 Hz, 1H), 7.31 (t, J = 8.0 Hz, 1H), 4.09 (q, J =7.2 Hz, 2H), 3.81 (s, 3H), 3.36 (t, J = 7.2 Hz, 2H), 2.97 (s, 3H), 2.92 (s, 3H), 2.29 (t, J =7.6 Hz, 2H), 1.63-1.58 (m, 4H), 1.42-1.34 (m, 8H), 1.23 (t, J =7.2 Hz, 3H); MS (EI) m/z = 633 (M⁺).

### Reference Example 8: Ethyl 10-(6-((4-(2-methoxy-3-(methylcarbamoyl)phenyl)amino)-5-(methylcarbamoyl)pyridin-2-yl)amino)nicotinamide)decanoate

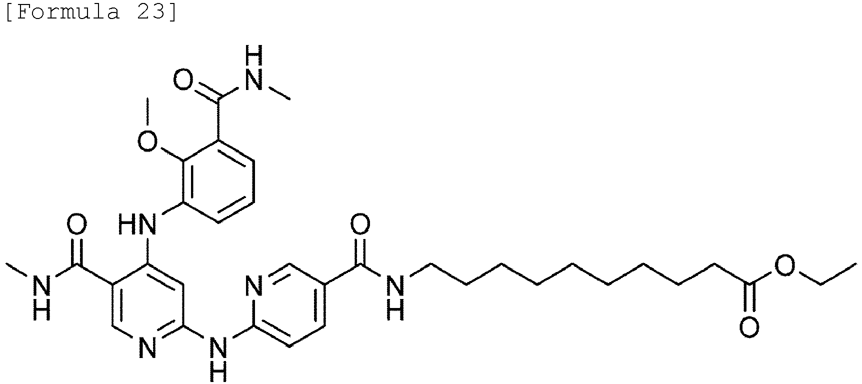

Ethyl 10-aminodecanoate hydrochloride (112 mg, 0.44 mmol) was added to a 6-((4-(2-methoxy-3-(methylcarbamoyl)anilino)-5-(methylcarbamoyl)-2-pyridyl)amino)pyridine-3-carboxylic acid (Reference Example 3) (100 mg, 0.22 mmol) solution in pyridine (2.0 mL), then 1-(3-dimethylaminopropyl)-3-ethylcarbodiimide hydrochloride (EDCI·HCl) (55.3 mg, 0.29 mmol) and 1-hydroxybenzotriazole (HOBt·H₂O) (6.80 mg, 0.04 mmol) were sequentially added thereto, followed by stirring at 50°C for 20 hours. The reaction solution was allowed to cool to room temperature, and mixed with a saturated sodium bicarbonate solution and chloroform. The mixture was stirred for 30 minutes, and the organic layer was separated therefrom. The aqueous layer was further subjected to extraction twice with chloroform. The organic layers were combined and dried over magnesium sulfate, followed by removal of the solvent under reduced pressure. The thus-obtained residue was mixed with water and 1.0 mol/L hydrochloric acid. The thus-precipitated solid was collected by filtration and mixed with diethyl ether, followed by stirring for 30 minutes and then filtration. The thus-obtained solid was dried under reduced pressure at 80°C to give the title compound (135 mg, 0.21 mmol, 94% yield).
¹H-NMR (d₄-MeOH 400 MHz) δ 8.81 (d, J = 2.4 Hz, 1H), 8.43 (s, 1H), 8.21 (dd, J = 8.8 Hz, 2.4 Hz, 1H), 7.67 (dd, J = 8.0 Hz, 1.6 Hz, 1H), 7.63 (dd, J = 8.0 Hz, 1.6 Hz, 1H), 7.35 (t, J = 8.4 Hz, 1H), 7.08 (d, J = 8.8 Hz, 1H), 6.65 (brs, 1H), 4.10 (q, J =7.2Hz, 2H), 3.82 (s, 3H), 3.38 (t, J = 7.2 Hz, 2H), 2.97 (s, 3H), 2.96 (s, 3H), 2.29 (t, J =7.2 Hz, 2H), 1.66-1.57 (m, 4H), 1.42-1.34 (m, 10H), 1.23 (t, J =7.2 Hz, 3H); MS (EI) m/z = 647 (M⁺).

### Reference Example 9: Ethyl 11-(6-((4-(2-methoxy-3-(methylcarbamoyl)phenyl)amino)-5-(methylcarbamoyl)pyridin-2-yl)amino)nicotinamide)undecanoate

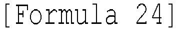

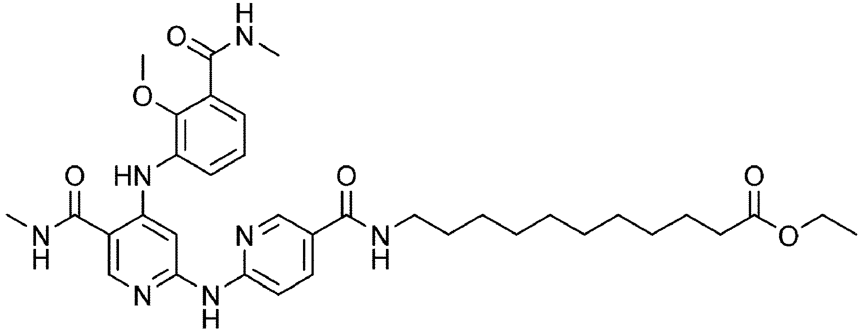

Using 6-((4-(2-methoxy-3-(methylcarbamoyl)anilino)-5-(methylcarbamoyl)-2-pyridyl)amino)pyridine-3-carboxylic acid (Reference Example 3) (100 mg, 0.22 mmol) and ethyl 11-aminoundecanoate hydrochloride (118 mg, 0.44 mmol), the same procedure as in Reference Example 4 was performed to give the title compound (230 mg, 0.35 mmol, 157 % yield). ¹H-NMR (d₄-MeOH 400 MHz) δ 8.66 (d, J = 2.0 Hz, 1H), 8.41 (s, 1H), 8.05 (dd, J = 8.8 Hz, 2.8 Hz, 1H), 7.90 (s, 1H), 7.72 (dd, J = 8.8 Hz, 1.6 Hz, 1H), 7.52 (dd, J = 8.0 Hz, 1.6 Hz, 1H), 7.36 (d, J = 8.8 Hz, 1H), 7.31 (t, J = 8.0 Hz, 1H), 4.13-4.06 (m, 2H), 3.81 (s, 3H), 3.36 (t, J = 7.6 Hz, 2H), 2.96 (s, 3H), 2.93 (s, 3H), 2.31-2.26 (m, 2H), 1.65-1.54 (m, 4H), 1.40-1.28 (m, 12H), 1.25-1.21 (m, 3H); MS (EI) m/z = 661 (M⁺).

### Reference Example 10: Methyl 12-(6-((4-(2-methoxy-3-(methylcarbamoyl)phenyl)amino)-5-(methylcarbamoyl)pyridin-2-yl)amino)nicotinamide)dodecanoate

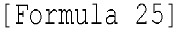

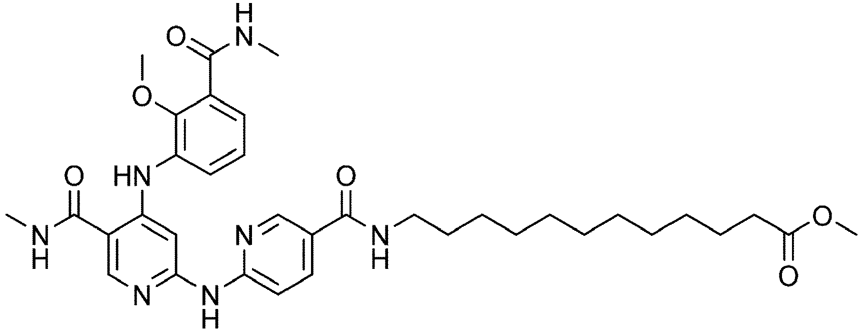

Using 6-((4-(2-methoxy-3-(methylcarbamoyl)anilino)-5-(methylcarbamoyl)-2-pyridyl)amino)pyridine-3-carboxylic acid (Reference Example 3) (100 mg, 0.22 mmol) and ethyl 12-aminododecanoate hydrochloride (118 mg, 0.44 mmol), the same procedure as in Reference Example 4 was performed to give the title compound (210 mg, 0.35 mmol, 143 % yield). ¹H-NMR (d₄-MeOH 400 MHz) δ 8.64 (d, J = 2.0 Hz, 1H), 8.40 (s, 1H), 8.04 (dd, J = 8.8 Hz, 2.8 Hz, 1H), 7.90 (s, 1H), 7.74 (dd, J = 8.0 Hz, 1.6 Hz, 1H), 7.51 (dd, J = 8.0 Hz, 1.6 Hz, 1H), 7.41 (d, J = 8.8 Hz, 1H), 7.32 (t, J = 8.0 Hz, 1H), 3.81 (s, 3H), 3.63 (s, 3H), 3.36 (t, J = 6.8 Hz, 2H), 2.97 (s, 3H), 2.93 (s, 3H), 2.30 (q, J = 6.8 Hz, 2H), 1.67-1.53 (m, 4H), 1.40-1.28 (m, 14H); MS (EI) m/z = 661 (M⁺).

### Reference Example 11: 4-(6-((4-(2-Methoxy-3-(methylcarbamoyl)phenyl)amino)-5-(methylcarbamoyl)pyridin-2-yl)amino)nicotinamide)butanoic acid

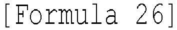

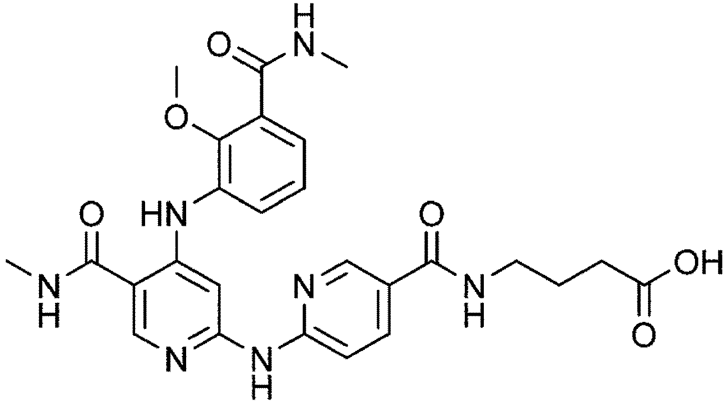

Ethyl 4-(6-((4-(2-methoxy-3-(methylcarbamoyl)phenyl)amino)-5-(methylcarbamoyl)pyridin-2-yl)amino)nicotinamide)butanoate (Reference Example 4) (125 mg, 0.22 mmol) was dissolved in a solvent mixture of ethanol (4.0 mL), tetrahydrofuran (THF) (4.0 mL) and purified water (4.0 mL), then 4.0 mol/L lithium hydroxide solution (1.1 mL, 4.44 mmol) was added thereto, followed by stirring for 3 hours at room temperature. Water was added to the reaction solution, which was then neutralized with 6.0 mol/L hydrochloric acid, followed by stirring for 2 hours. The thus-precipitated solid was collected by filtration and dried under reduced pressure at 80°C for 2 hours to give the title compound (79.0 mg, 0.15 mmol, 66% yield).
¹H-NMR (d₄-MeOH 400 MHz) δ 8.65 (d, J = 1.6 Hz, 1H), 8.40 (s, 1H), 8.05 (dd, J = 8.8 Hz, 2.4 Hz, 1H), 7.78 (brs, 1H), 7.73 (dd, J = 8.4 Hz, 1.6 Hz, 1H), 7.51 (dd, J = 8.0 Hz, 1.6 Hz, 1H), 7.39 (d, J = 8.8 Hz, 1H), 7.32 (t, J = 8.4 Hz, 1H), 3.81 (s, 3H), 3.42 (t, J = 6.8 Hz, 2H), 2.97 (s, 3H), 2.93 (s, 3H), 2.38 (t, J = 7.2 Hz, 2H), 1.95-1.88 (m, 2H); MS (EI) m/z = 535 (M⁺).

### Reference Example 12: 6-(6-((4-(2-Methoxy-3-(methylcarbamoyl)phenyl)amino)-5-(methylcarbamoyl)pyridin-2-yl)amino)nicotinamide)hexanoic acid

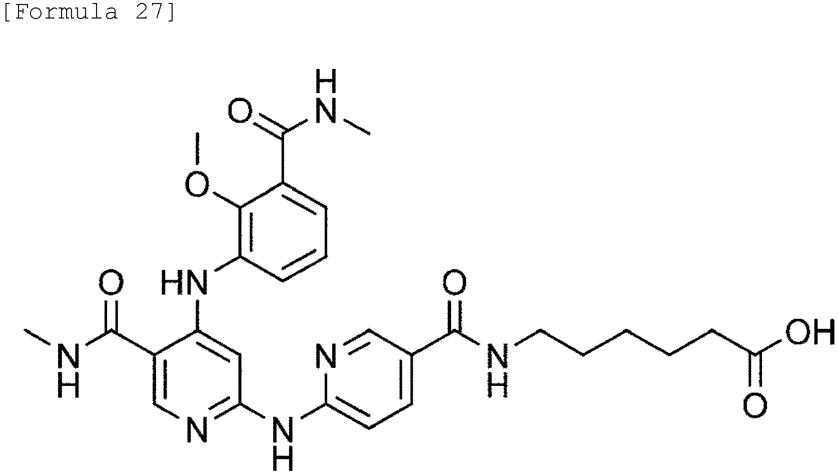

Using ethyl 6-(6-((4-(2-methoxy-3-(methylcarbamoyl)phenyl)amino)-5-(methylcarbamoyl)pyridin-2-yl)amino)nicotinamide)hexanoate (Reference Example 5) (131 mg, 0.22 mmol), the same procedure as in Reference Example 11 was performed to give the title compound (119 mg, 0.21 mmol, 95 % yield).
¹H-NMR (d₄-MeOH 400 MHz) δ 8.64 (d, J = 2.0 Hz, 1H), 8.40 (s, 1H), 8.04 (dd, J = 8.8 Hz, 2.4 Hz, 1H), 7.80 (s, 1H), 7.73 (dd, J = 8.0 Hz, 1.6 Hz, 1H), 7.51 (dd, J = 7.6 Hz, 2.0 Hz, 1H), 7.41 (d, J = 8.8 Hz, 1H), 7.31 (t, J = 8.0 Hz, 1H), 3.81 (s, 3H), 3.37 (t, J = 6.8 Hz, 2H), 2.96 (s, 3H), 2.92 (s, 3H), 2.31 (t, J = 7.2 Hz, 2H), 1.70-1.60 (m, 4H), 1.47-1.39 (m, 2H); MS (EI) m/z = 563 (M⁺).

### Reference Example 13: 8-(6-((4-(2-Methoxy-3-(methylcarbamoyl)phenyl)amino)-5-(methylcarbamoyl)pyridin-2-yl)amino)nicotinamide)octanoic acid

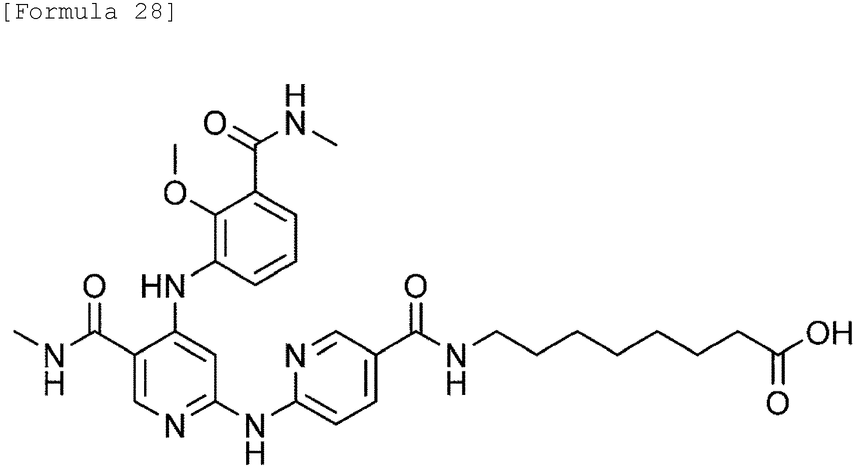

Using ethyl 8-(6-((4-(2-methoxy-3-(methylcarbamoyl)phenyl)amino)-5-(methylcarbamoyl)pyridin-2-yl)amino)nicotinamide)octanoate (Reference Example 6) (146 mg, 0.24 mmol), the same procedure as in Reference Example 11 was performed to give the title compound (125 mg, 0.21 mmol, 90% yield).
¹H-NMR (d₄-MeOH 400 MHz) δ 8.64 (d, J = 2.0 Hz, 1H), 8.40 (s, 1H), 8.03 (dd, J = 8.8 Hz, 2.4 Hz, 1H), 7.82 (s, 1H), 7.73 (dd, J = 8.4 Hz, 1.6 Hz, 1H), 7.50 (dd, J = 8.0 Hz, 1.6 Hz, 1H), 7.42 (d, J = 8.8 Hz, 1H), 7.31 (t, J = 8.0 Hz, 1H), 3.81 (s, 3H), 3.36 (t, J = 6.8 Hz, 2H), 2.96 (s, 3H), 2.92 (s, 3H), 2.27 (t, J = 7.6 Hz, 2H), 1.63-1.59 (m, 4H), 1.41-1.35 (m, 6H); MS (EI) m/z = 591 (M⁺).

### Reference Example 14: 9-(6-((4-(2-Methoxy-3-(methylcarbamoyl)phenyl)amino)-5-(methylcarbamoyl)pyridin-2-yl)amino)nicotinamide)nonanoic acid

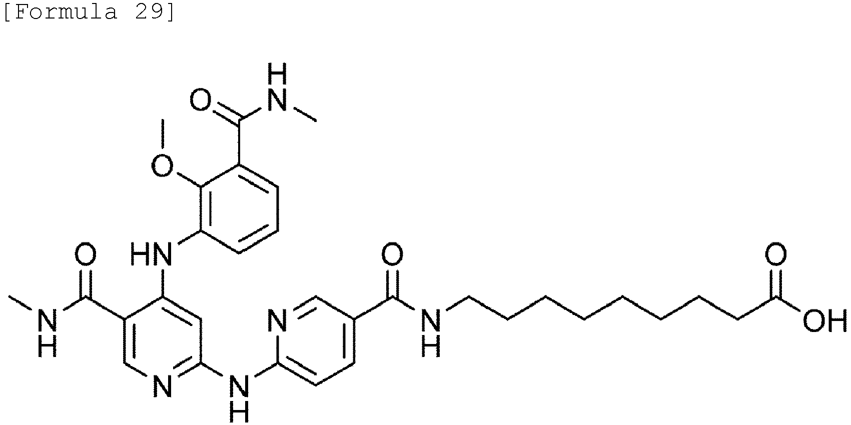

Using ethyl 9-(6-((4-(2-methoxy-3-(methylcarbamoyl)phenyl)amino)-5-(methylcarbamoyl)pyridin-2-yl)amino)nicotinamide)nonanoate (Reference Example 7) (146 mg, 0.18 mmol), the same procedure as in Reference Example 11 was performed to give the title compound (105 mg, 0.17 mmol, 94% yield).
¹H-NMR (d₄-MeOH 400 MHz) δ 8.80 (d, J = 2.4 Hz, 1H), 8.43 (s, 1H), 8.20 (dd, J = 8.8 Hz, 2.4 Hz, 1H), 7.67-7.61 (m, 2H), 7.35 (t, J = 8.4 Hz, 1H), 7.10 (d, J = 8.8 Hz, 1H), 6.74 (brs, 1H), 3.82 (s, 3H), 3.41-3.36 (m, 2H), 2.97 (d, J = 5.2 Hz, 3H), 2.96 (s, 3H), 2.27 (t, J =7.6 Hz, 2H), 1.65-1.57 (m, 4H), 1.42-1.29 (m, 8H); MS (EI) m/z = 605 (M⁺).

### Reference Example 15: 10-(6-((4-(2-Methoxy-3-(methylcarbamoyl)phenyl)amino)-5-(methylcarbamoyl)pyridin-2-yl)amino)nicotinamide)decanoic acid

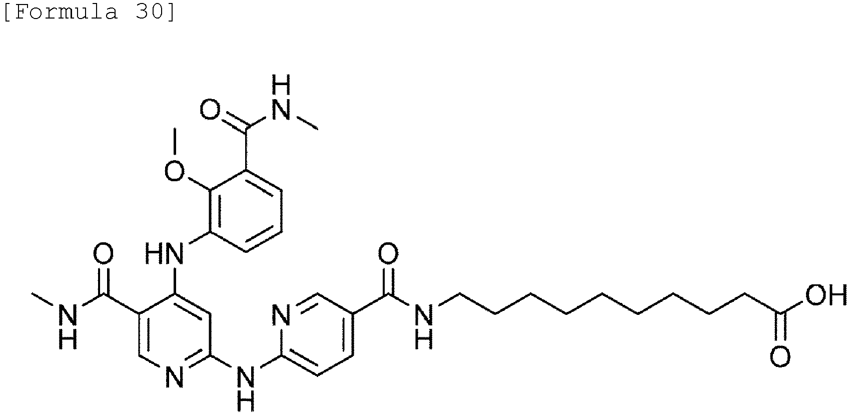

Using ethyl 10-(6-((4-(2-methoxy-3-(methylcarbamoyl)phenyl)amino)-5-(methylcarbamoyl)pyridin-2-yl)amino)nicotinamide)decanoate (Reference Example 8) (120 mg, 0.19 mmol), the same procedure as in Reference Example 11 was performed to give the title compound (108 mg, 0.17 mmol, 94% yield).
¹H-NMR (d₆-DMSO 400 MHz) δ 11.9 (brs, 1H), 10.9 (s, 1H), 9.10 (d, J = 4.0 Hz, 1H), 8.79 (d, J = 2.0 Hz, 1H), 8.63 (t, J = 5.6 Hz, 1H), 8.55 (s, 1H), 8.32-8.31 (m, 1H), 8.26 (dd, J = 8.8 Hz, 2.4 Hz, 1H), 7.68 (m, 1H), 7.59 (dd, J = 7.6 Hz, 1.6 Hz, 1H), 7.45 (dd, J = 7.6 Hz, 1.6 Hz, 1H), 7.30 (t, J = 8.0 Hz, 1H), 7.22 (d, J = 8.8 Hz, 1H), 6.94 (brs, 1H), 3.73 (s, 3H), 3.28-3.23 (m, 2H), 2.83 (d, J = 4.4 Hz, 3H), 2.81 (d, J = 4.4 Hz, 3H), 2.22-2.14 (m, 2H), 1.54-1.46 (m, 4H), 1.29-1.23 (m, 10H); MS (EI) m/z = 619 (M⁺).

### Reference Example 16: 11-(6-((4-(2-Methoxy-3-(methylcarbamoyl)phenyl)amino)-5-(methylcarbamoyl)pyridin-2-yl)amino)nicotinamide)undecanoic acid

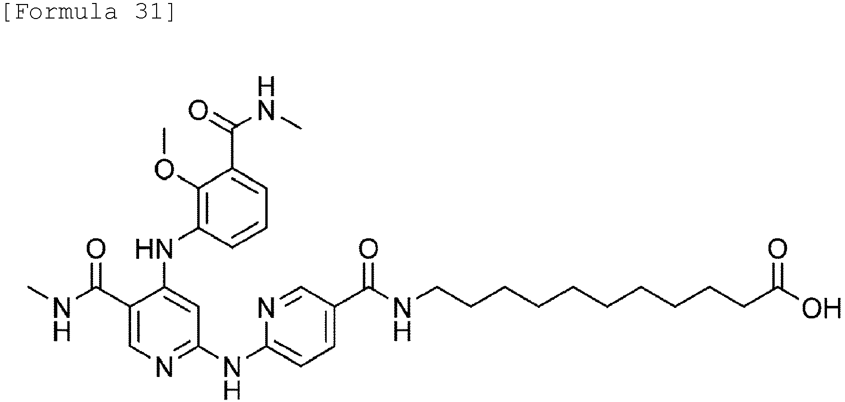

Using ethyl 11-(6-((4-(2-methoxy-3-(methylcarbamoyl)phenyl)amino)-5-(methylcarbamoyl)pyridin-2-yl)amino)nicotinamide)undecanoate (Reference Example 9) (146 mg, 0.22 mmol), the same procedure as in Reference Example 11 was performed to give the title compound (79.0 mg, 0.12 mmol, 57% yield).
¹H-NMR (d₄-MeOH 400 MHz) δ 8.63 (d, J = 2.0 Hz, 1H), 8.40 (s, 1H), 8.03 (dd, J = 8.8 Hz, 2.4 Hz, 1H), 7.87 (s, 1H), 7.74 (dd, J = 8.0 Hz, 1.6 Hz, 1H), 7.50 (dd, J = 8.0 Hz, 1.6 Hz, 1H), 7.45 (d, J = 8.8 Hz, 1H), 7.31 (t, J = 8.0 Hz, 1H), 3.81 (s, 3H), 3.36 (t, J = 6.8 Hz, 2H), 2.97 (s, 3H), 2.92 (s, 3H), 2.24 (t, J = 7.6 Hz, 2H), 1.63-1.57 (m, 4H), 1.40-1.28 (m, 12H); MS (EI) m/z = 633 (M⁺).

### Reference Example 17: 12-(6-((4-(2-Methoxy-3-(methylcarbamoyl)phenyl)amino)-5-(methylcarbamoyl)pyridin-2-yl)amino)nicotinamide)dodecanoic acid

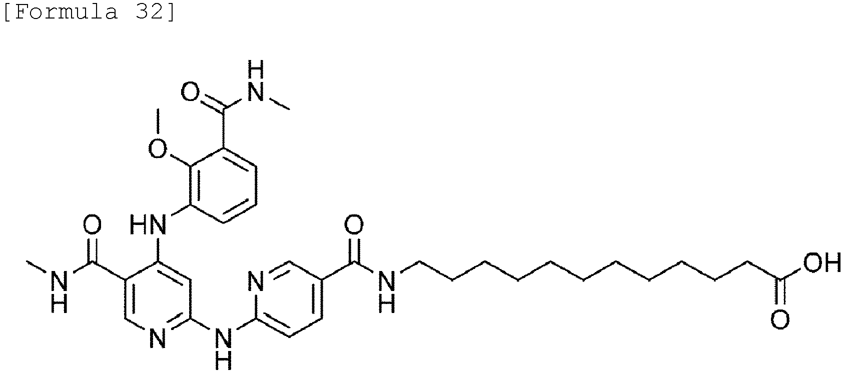

Using methyl 12-(6-((4-(2-methoxy-3-(methylcarbamoyl)phenyl)amino)-5-(methylcarbamoyl)pyridin-2-yl)amino)nicotinamide)dodecanoate (Reference Example 10) (146 mg, 0.22 mmol), the same procedure as in Reference Example 11 was performed to give the title compound (94.0 mg, 0.15 mmol, 66% yield).
¹H-NMR (d₄-MeOH 400 MHz) δ 8.64 (d, J = 1.6 Hz, 1H), 8.41 (s, 1H), 8.04 (dd, J = 8.8 Hz, 2.8 Hz, 1H), 7.75 (brs, 1H), 7.74 (dd, J = 8.4 Hz, 1.6 Hz, 1H), 7.51 (dd, J = 8.0 Hz, 1.6 Hz, 1H), 7.41 (d, J = 8.8 Hz, 1H), 7.32 (t, J = 8.0 Hz, 1H), 3.81 (s, 3H), 3.36 (t, J = 6.8 Hz, 2H), 2.97 (s, 3H), 2.93 (s, 3H), 2.25 (t, J = 7.6 Hz, 2H), 1.68-1.56 (m, 4H), 1.41-1.28 (m, 14H); MS (EI) m/z = 647 (M⁺).

### Example 1: 6-((5-((4-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-4-oxobutyryl)carbamoyl)pyridin-2-yl)amino)-4-((2-methoxy-3-(methylcarbamoyl)phenyl)amino)-N-methylnicotinamide

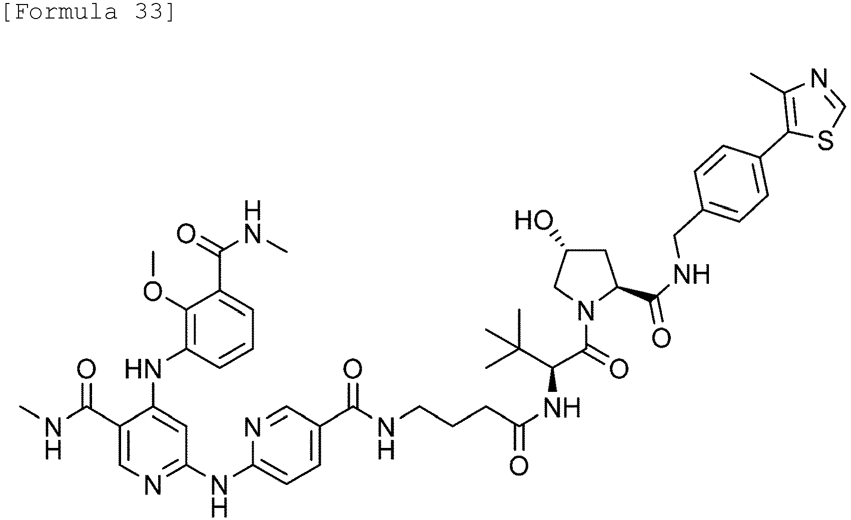

To a 4-(6-((4-(2-methoxy-3-(methylcarbamoyl)phenyl)amino)-5-(methylcarbamoyl)pyridin-2-yl)amino)nicotinamide)butanoic acid (Reference Example 11) (78.0 mg, 0.15 mmol) solution in N,N-dimethylformamide (DMF) (1.0 mL) were added (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (71.4 mg, 0.15 mmol) and N,N-diisopropylethylamine (DIPEA) (113 mg, 0.87 mmol), followed by cooling in an ice bath. Then, O-(7-azabenzotriazol-1-yl)-N,N,N',N'-tetramethyluronium hexafluorophosphate (HATU) (83.1 mg, 0.22 mmol) was added to the reaction solution at the same temperature, followed by stirring for 10 minutes. The reaction solution was heated to room temperature, stirred for 26 hours, mixed with a saturated sodium bicarbonate solution and a solvent mixture of ethyl acetate/methanol, and stirred for 30 minutes, followed by separation of the organic layer. The aqueous layer was further subjected to extraction twice with a solvent mixture of ethyl acetate/methanol. The organic layers were combined, washed three times with water, and dried over magnesium sulfate, followed by removal of the solvent under reduced pressure. The thus-obtained residue was purified by silica gel column chromatography (methanol/chloroform = 10%) and the solvent was distilled off under reduced pressure. Diethyl ether was added to the resulting solid, which was then collected by filtration to give the title compound (130 mg, 0.14 mmol, 94% yield).
¹H-NMR (d₄-MeOH 400 MHz) δ 8.85 (s, 1H), 8.68 (d, J = 2.0 Hz, 1H), 8.40 (s, 1H), 8.07 (dd, J = 8.8 Hz, 2.4 Hz, 1H), 7.99 (d, J = 8.8 Hz, 1H), 7.71 (dd, J = 8.4 Hz, 1.6 Hz, 1H), 7.53 (d, J = 7.2 Hz, 1H), 7.45 (d, J = 8.4 Hz, 2H), 7.38 (d, J = 8.4 Hz, 2H), 7.38-7.37 (m, 1H), 7.31 (t, J = 8.0 Hz, 1H), 4.63-4.50 (m, 4H), 4.34 (dd, J = 15.6 Hz, 4.4 Hz, 1H), 3.92 (d, J = 10.8 Hz, 1H), 3.83-3.79 (m, 1H), 3.80 (s, 3H), 3.43-3.37 (m, 2H), 2.96 (s, 3H), 2.93 (s, 3H), 2.44 (s, 3H), 2.37 (t, J = 7.6 Hz, 2H), 2.23-2.19 (m, 1H), 2.12-2.08 (m, 1H), 1.94-1.86 (m, 2H), 1.05 (s, 9H); MS (ESI) m/z = 948.4260 (MH⁺).

### Example 2: 6-((5-((6-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-6-oxohexyl)carbamoyl)pyridin-2-yl)amino)-4-((2-methoxy-3-(methylcarbamoyl)phenyl)amino)-N-methylnicotinamide

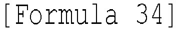

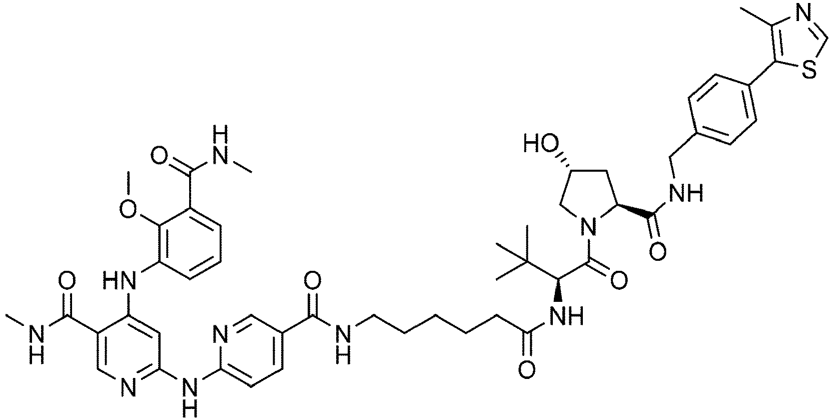

Using 6-(6-((4-(2-methoxy-3-(methylcarbamoyl)phenyl)amino)-5-(methylcarbamoyl)pyridin-2-yl)amino)nicotinamide)hexanoic acid (Reference Example 12) (95.0 mg, 0.17 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (94.6 mg, 0.20 mmol), the same procedure as in Example 1 was performed to give the title compound (48.0 mg, 0.05 mmol, 29% yield).
¹H-NMR (d₄-MeOH 400 MHz) δ 8.85 (s, 1H), 8.63 (d, J = 2.0 Hz, 1H), 8.40 (s, 1H), 8.02 (dd, J = 8.8 Hz, 2.4 Hz, 1H), 7.91 (brs, 1H), 7.73 (dd, J = 8.0 Hz, 1.2 Hz, 1H), 7.45 (dd, J = 8.0 Hz, 1.6 Hz, 1H), 7.44 (d, J = 8.8 Hz, 2H), 7.39-7.37 (m, 3H), 7.30 (t, J = 8.0 Hz, 1H), 4.62-4.48 (m, 4H), 4.33 (d, J = 15.6 Hz, 1H), 3.90 (d, J = 11.2 Hz, 1H), 3.80 (s, 3H), 3.80-3.78 (m, 1H), 3.41-3.30 (m, 2H), 2.96 (s, 3H), 2.92 (s, 3H), 2.45 (s, 3H), 2.34-2.18 (m, 3H), 2.10-1.99 (m, 1H), 1.70-1.59 (m, 4H), 1.46-1.36 (m, 2H), 1.01 (s, 9H); MS (ESI) m/z = 976.4578 (MH⁺).

### Example 3: 6-((5-((8-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-8-oxooctyl)carbamoyl)pyridin-2-yl)amino)-4-((2-methoxy-3-(methylcarbamoyl)phenyl)amino)-N-methylnicotinamide

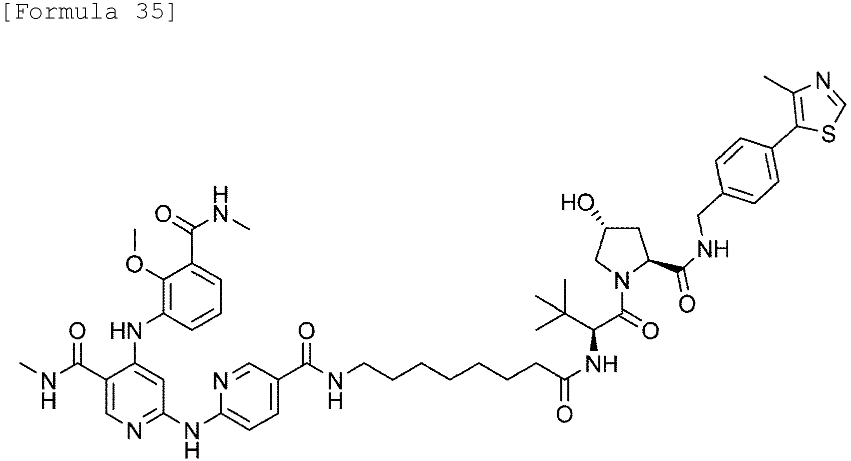

Using 8-(6-((4-(2-methoxy-3-(methylcarbamoyl)phenyl)amino)-5-(methylcarbamoyl)pyridin-2-yl)amino)nicotinamide)octanoic acid (Reference Example 13) (116 mg, 0.20 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (110 mg, 0.24 mmol), the same procedure as in Example 1 was performed to give the title compound (168 mg, 0.17 mmol, 85% yield).
¹H-NMR (d₄-MeOH 400 MHz) δ 8.85 (s, 1H), 8.64 (d, J = 2.0 Hz, 1H), 8.40 (s, 1H), 8.03 (dd, J = 8.8 Hz, 2.4 Hz, 1H), 7.85-7.82 (m, 1H), 7.72 (dd, J = 8.0 Hz, 1.6 Hz, 1H), 7.51 (dd, J = 8.0 Hz, 1.6 Hz, 1H), 7.44 (d, J = 8.4 Hz, 2H), 7.40-7.37 (m, 3H), 7.30 (t, J = 8.0 Hz, 1H), 4.64-4.48 (m, 4H), 4.34 (d, J = 16.0 Hz, 1H), 3.90 (d, J = 10.8 Hz, 1H), 3.80 (s, 3H), 3.78-3.69 (m, 1H), 3.38-3.34 (m, 2H), 2.96 (s, 3H), 2.93 (s, 3H), 2.45 (s, 3H), 2.34-2.18 (m, 3H), 2.10-2.03 (m, 1H), 1.68-1.56 (m, 4H), 1.42-1.30 (m, 6H), 1.02 (s, 9H); MS (ESI) m/z = 1004.4923 (MH⁺).

### Example 4: 6-((5-((9-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-9-oxononyl)carbamoyl)pyridin-2-yl)amino)-4-((2-methoxy-3-(methylcarbamoyl)phenyl)amino)-N-methylnicotinamide

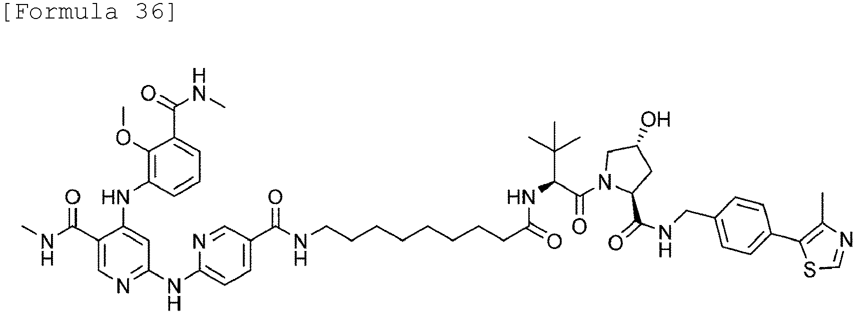

Using 9-(6-((4-(2-methoxy-3-(methylcarbamoyl)phenyl)amino)-5-(methylcarbamoyl)pyridin-2-yl)amino)nicotinamide)nonanoic acid (Reference Example 14) (100 mg, 0.17 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (92.5 mg, 0.20 mmol), the same procedure as in Example 1 was performed to give the title compound (92.0 mg, 0.09 mmol, 55% yield).
¹H-NMR (d₄-MeOH 400 MHz) δ 8.85 (s, 1H), 8.62 (d, J = 2.0 Hz, 1H), 8.40 (s, 1H), 8.01 (dd, J = 8.8 Hz, 2.4 Hz, 1H), 7.84 (d, J = 8.8 Hz, 1H), 7.74 (dd, J = 8.0 Hz, 1.6 Hz, 1H), 7.49 (dd, J = 8.0 Hz, 1.6 Hz, 1H), 7.44 (d, J = 7.6 Hz, 2H), 7.41-7.37 (m, 3H), 7.30 (t, J = 8.0 Hz, 1H), 4.64-4.49 (m, 4H), 4.34 (d, J = 15.6 Hz, 1H), 3.90 (d, J = 11.2 Hz, 1H), 3.80 (s, 3H), 3.80-3.77 (m, 1H), 3.37-3.30 (m, 2H), 2.96 (s, 3H), 2.92 (s, 3H), 2.45 (s, 3H), 2.33-2.19 (m, 3H), 2.10-2.04 (m, 1H), 1.65-1.55 (m, 4H), 1.40-1.29 (m, 8H), 1.02 (s, 9H); MS (ESI) m/z = 1018.5030 (MH⁺).

### Example 5: 6-((5-((10-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-10-oxodecyl)carbamoyl)pyridin-2-yl)amino)-4-((2-methoxy-3-(methylcarbamoyl)phenyl)amino)-N-methylnicotinamide

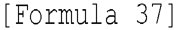

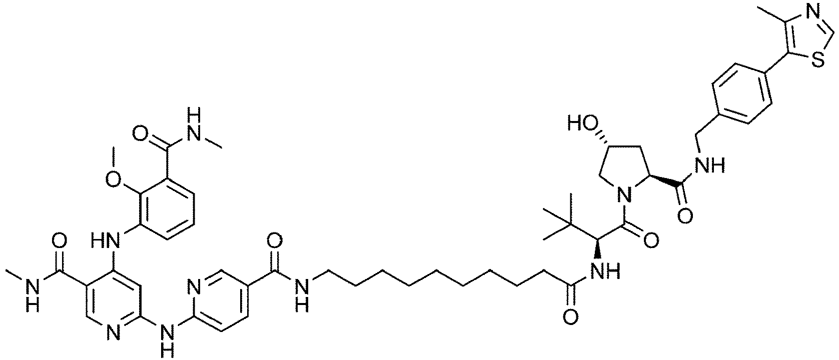

Using 10-(6-((4-(2-methoxy-3-(methylcarbamoyl)phenyl)amino)-5-(methylcarbamoyl)pyridin-2-yl)amino)nicotinamide)decanoic acid (Reference Example 15) (80.0 mg, 0.13 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (72.4 mg, 0.15 mmol), the same procedure as in Example 1 was performed to give the title compound (83.0 mg, 0.08 mmol, 62% yield).
¹H-NMR (d₄-MeOH 400 MHz) δ 8.85 (s, 1H), 8.65 (d, J = 2.0 Hz, 1H), 8.40 (s, 1H), 8.04 (dd, J = 8.8 Hz, 2.8 Hz, 1H), 7.80 (d, J = 8.8 Hz, 1H), 7.72 (dd, J = 7.6 Hz, 1.6 Hz, 1H), 7.52 (dd, J = 7.6 Hz, 1.6 Hz, 1H), 7.44 (d, J = 8.4 Hz, 2H), 7.42-7.37 (m, 3H), 7.31 (t, J = 7.6 Hz, 1H), 4.64-4.49 (m, 4H), 4.34 (dd, J = 15.6 Hz, 4.4 Hz, 1H), 3.90 (d, J = 11.2 Hz, 1H), 3.81 (s, 3H), 3.78-3.71 (m, 1H), 3.36 (t, J = 7.6 Hz, 2H), 2.96 (s, 3H), 2.93 (s, 3H), 2.45 (s, 3H), 2.33-2.18 (m, 3H), 2.11-2.04 (m, 1H), 1.63-1.55 (m, 4H), 1.37-1.28 (m, 10H), 1.02 (s, 9H); MS (ESI) m/z = 1032.5155 (MH⁺).

### Example 6: 6-((5-((11-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-11-oxoundecyl)carbamoyl)pyridin-2-yl)amino)-4-((2-methoxy-3-(methylcarbamoyl)phenyl)amino)-N-methylnicotinamide

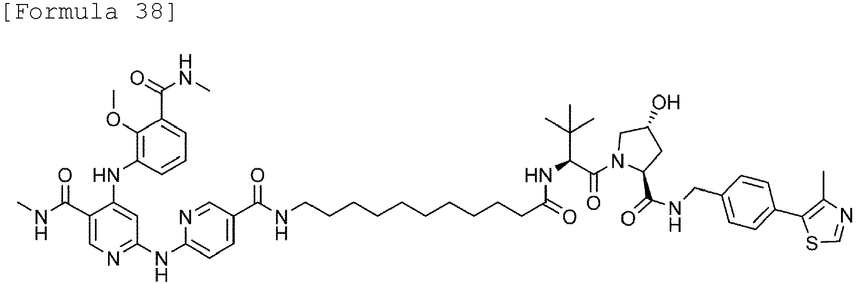

Using 11-(6-((4-(2-methoxy-3-(methylcarbamoyl)phenyl)amino)-5-(methylcarbamoyl)pyridin-2-yl)amino)nicotinamide)undecanoic acid (Reference Example 16) (74.7 mg, 0.12 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (66.0 mg, 0.14 mmol), the same procedure as in Example 1 was performed to give the title compound (82.0 mg, 0.08 mmol, 67% yield).
¹H-NMR (d₄-MeOH 400 MHz) δ 8.86 (s, 1H), 8.63 (d, J = 2.0 Hz, 1H), 8.40 (s, 1H), 8.02 (dd, J = 8.8 Hz, 2.8 Hz, 1H), 7.84 (d, J = 8.8 Hz, 2H), 7.74 (dd, J = 8.4 Hz, 1.6 Hz, 1H), 7.49 (dd, J = 8.0 Hz, 1.6 Hz, 1H), 7.44 (d, J = 8.0 Hz, 2H), 7.42-7.38 (m, 3H), 7.30 (t, J = 8.0 Hz, 1H), 4.64-4.49 (m, 4H), 4.33 (d, J = 15.6 Hz, 1H), 3.90 (d, J = 11.2 Hz, 1H), 3.80 (s, 3H), 3.80-3.77 (m, 1H), 3.37-3.34 (m, 2H), 2.96 (s, 3H), 2.92 (s, 3H), 2.46 (s, 3H), 2.32-2.17 (m, 3H), 2.10-2.04 (m, 1H), 1.65-1.54 (m, 4H), 1.41-1.28 (m, 12H), 1.02 (s, 9H); MS (ESI) m/z = 1046.5398 (MH⁺).

### Example 7: 6-((5-((12-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-12-oxododecyl)carbamoyl)pyridin-2-yl)amino)-4-((2-methoxy-3-(methylcarbamoyl)phenyl)amino)-N-methylnicotinamide

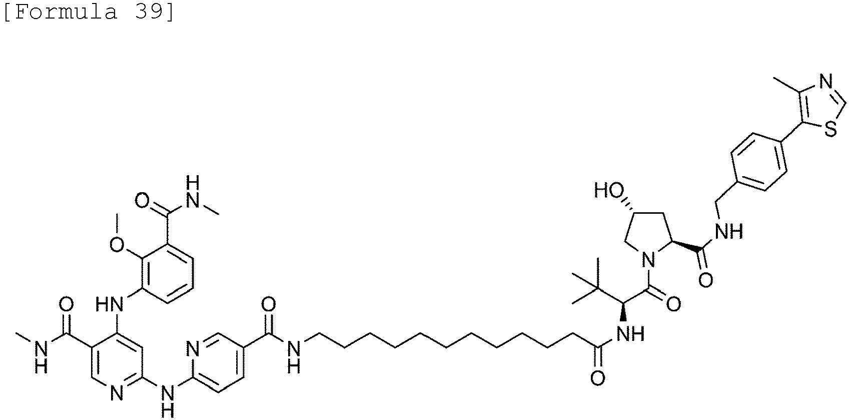

Using 12-(6-((4-(2-methoxy-3-(methylcarbamoyl)phenyl)amino)-5-(methylcarbamoyl)pyridin-2-yl)amino)nicotinamide)dodecanoic acid (Reference Example 17) (96.1 mg, 0.15 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (83.1 mg, 0.18 mmol), the same procedure as in Example 1 was performed to give the title compound (45.0 mg, 0.04 mmol, 29% yield).
¹H-NMR (d₄-MeOH 400 MHz) δ 8.86 (s, 1H), 8.63 (d, J = 2.0 Hz, 1H), 8.40 (s, 1H), 8.02 (dd, J = 8.8 Hz, 2.8 Hz, 1H), 7.86-7.83 (m, 1H), 7.74 (dd, J = 8.4 Hz, 1.6 Hz, 1H), 7.49 (dd, J = 7.6 Hz, 2.0 Hz, 1H), 7.46-7.43 (m, 3H), 7.39 (d, J = 8.4 Hz, 2H), 7.30 (t, J = 7.6 Hz, 1H), 4.64-4.49 (m, 4H), 4.33 (d, J = 15.6 Hz, 1H), 3.90 (d, J = 11.2 Hz, 1H), 3.81 (s, 3H), 3.81-3.79 (m, 1H), 3.36 (t, J = 7.2 Hz, 2H), 2.96 (s, 3H), 2.92 (s, 3H), 2.45 (s, 3H), 2.28-2.18 (m, 3H), 2.09-2.04 (m, 1H), 1.64-1.57 (m, 4H), 1.41-1.25 (m, 14H), 1.03 (s, 9H); MS (ESI) m/z = 1061.5621 (MH⁺).

The synthesis scheme of Example 8 is shown below.

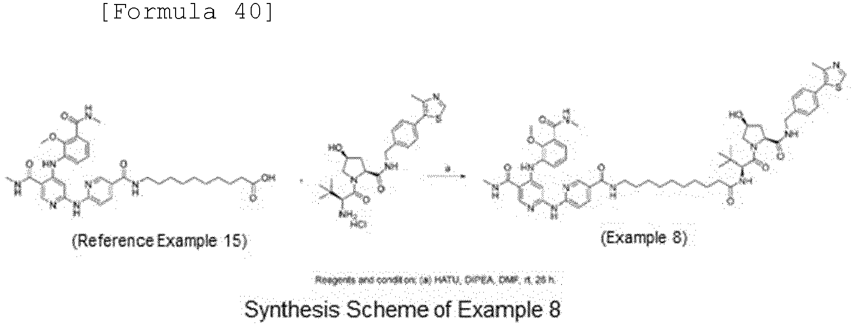

### Example 8: 6-((5-((10-(((S)-1-((2S,4S)-4-Hydroxy-2-((4-(4-methylthiazol-5-yl)benzyl)carbamoyl)pyrrolidin-1-yl)-3,3-dimethyl-1-oxobutan-2-yl)amino)-10-oxodecyl)carbamoyl)pyridin-2-yl)amino)-4-((2-methoxy-3-(methylcarbamoyl)phenyl)amino)-N-methylnicotinamide

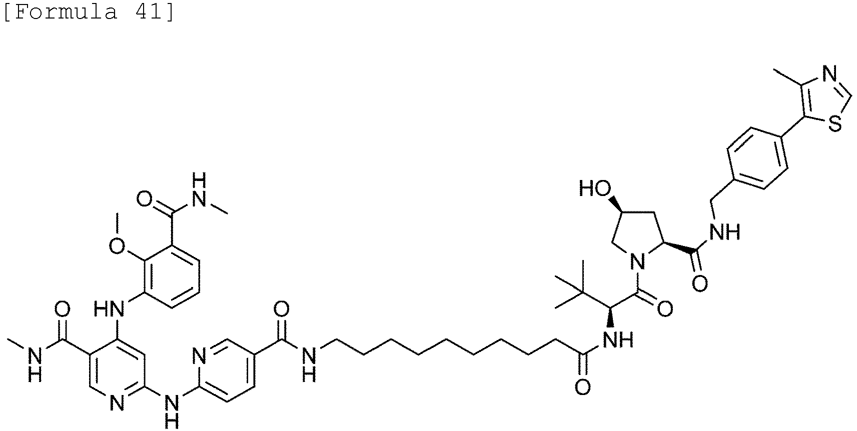

Using 10-(6-((4-(2-methoxy-3-(methylcarbamoyl)phenyl)amino)-5-(methylcarbamoyl)pyridin-2-yl)amino)nicotinamide)decanoic acid (Reference Example 15) (123 mg, 0.20 mmol) and (2S,4S)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in J. Med Chem. 2018, 61, 583.) (111 mg, 0.24 mmol), the same procedure as in Example 1 was performed to give the title compound (117 mg, 0.11 mmol, 57% yield).
¹H-NMR (d₄-MeOH 400 MHz) δ 8.86 (s, 1H), 8.62 (brs, 1H), 8.40 (brs, 1H), 8.02 (d, J = 7.2 Hz, 1H), 7.87 (d, J = 8.8 Hz, 1H), 7.74 (dd, J = 8.0 Hz, 1.2 Hz, 1H), 7.49 (dd, J = 8.0 Hz, 1.2 Hz, 1H), 7.44-7.42 (m, 3H), 7.39 (d, J = 8.0 Hz, 2H), 7.30 (t, J = 8.0 Hz, 1H), 4.56-4.48 (m, 3H), 4.40-4.34 (m, 2H), 4.03 (dd, J = 10.8 Hz, 5.2 Hz, 1H), 3.80 (s, 3H), 4.37 (dd, J = 10.8 Hz, 3.6 Hz, 1H), 3.35 (t, J = 7.6 Hz, 2H), 2.96 (s, 3H), 2.92 (s, 3H), 2.45 (s, 3H), 2.42-2.38 (m, 1H), 2.30-2.15 (m, 2H), 2.00-1.94 (m, 1H), 1.65-1.52 (m, 4H), 1.40-1.25 (m, 10H), 1.03 (s, 9H); MS (ESI) m/z = 1032.3 (MH⁺).

The synthesis scheme of Example 9 is shown below.

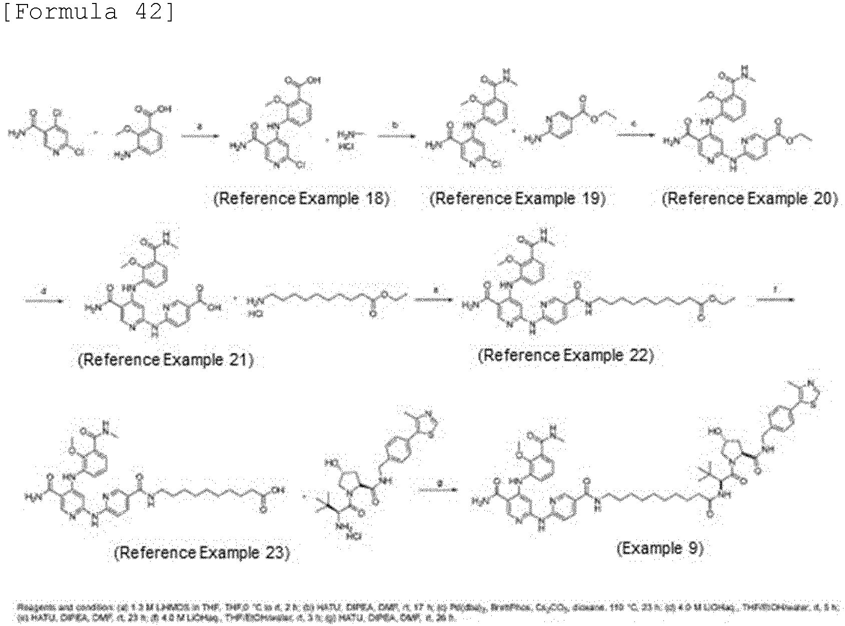

### Synthesis Scheme of Example 9

### Reference Example 18: 3-((5-Carbamoyl-2-chloropyridin-4-yl) amino)2-methoxybenzoic acid

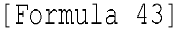

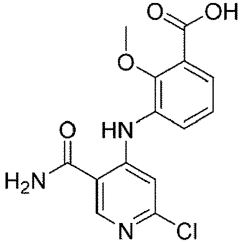

To a 4,6-dichloropyridine-3-carboxamide (2.02 g, 10.6 mmol) solution in N,N-dimethylacetamide (DMF) (7.0 mL) was added 3-amino-2-methoxybenzoic acid (2.12 g, 12.6 mmol), followed by cooling in an ice bath under a nitrogen atmosphere and then stirring for 30 min. To the reaction solution, a lithium bis(trimethylsilyl)amide (LiHMDS) solution in tetrahydrofuran (THF) (32.5 mL, 42.3 mmol) prepared at 1.30 mol/L was added dropwise over 8 minutes at the same temperature, followed by heating to room temperature and then stirring for 1 hour. The reaction solution was cooled in an ice bath, then slowly mixed with ice-cold water (50 mL), followed by neutralization with 6.0 mol/L hydrochloric acid and then stirring overnight. The thus-precipitated solid was collected by filtration, washed with water and then diethyl ether, and dried under reduced pressure at 80°C for 2 hours to give the title compound (2.99 g, 9.29 mmol, 88% yield).
¹H-NMR (d₆-DMSO 400 MHz) δ 13.1 (s, 1H),10.9 (s, 1H), 8.60 (s, 1H), 8.35 (brs, 1H), 7.80 (brs, 1H), 7.66 (dd, J = 8.0 Hz, 1.6 Hz, 1H), 7.52 (dd, J = 8.0 Hz, 1.6 Hz, 1H), 7.26 (t, J = 8.0 Hz, 1H), 6.90 (s, 1H), 3.72 (s, 3H); MS (EI) m/z = 321 (M⁺).

### Reference Example 19: 6-Chloro-4-((2-methoxy-3-(methylcarbamoyl)phenyl)amino)nicotinamide

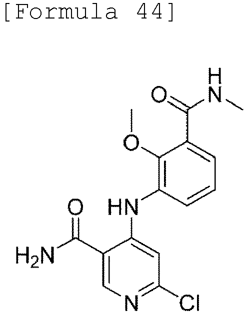

Using 3-((5-carbamoyl-2-chloropyridin-4-yl)amino)-2-methoxybenzoic acid (Reference Example 18) (1.50 g, 4.66 mmol), the same procedure as in Reference Example 1 was performed to give the title compound (1.23 g, 3.67 mmol, 79% yield).
¹H-NMR (d₆-DMSO 400 MHz) δ 10.8 (s, 1H), 8.60 (s, 1H), 8.35 (brs, 1H), 8.27 (dd, J = 8.8 Hz, 4.4 Hz, 1H), 7.80 (brs, 1H), 7.56 (dd, J = 8.0 Hz, 1.6 Hz, 1H), 7.36 (dd, J = 8.0 Hz, 1.6 Hz, 1H), 7.24 (t, J = 8.0 Hz, 1H), 6.88 (s, 1H), 3.68 (s, 3H), 2.79 (d, J = 4.4 Hz, 3H); MS (EI) m/z = 334 (M⁺).

### Reference Example 20: Ethyl-6-((5-carbamoyl-4-(2-methoxy-3-(methylcarbamoyl)anilino)-2-pyridyl)amino)pyridine-3-carboxylate

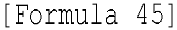

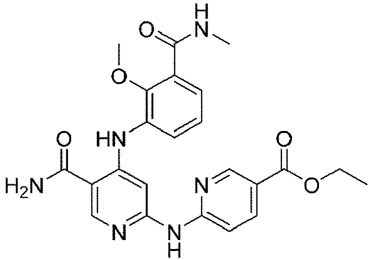

Using 6-chloro-4-((2-methoxy-3-(methylcarbamoyl)phenyl)amino)nicotinamide (Reference Example 19) (1.40 g, 4.18 mmol), the same procedure as in Reference Example 2 was performed to give the title compound (1.07 g, 2.30 mmol, 55% yield).
¹H-NMR (d₆-DMSO 400 MHz) δ 11.0 (s, 1H), 10.3 (s, 1H), 8.70 (d, J = 2.4 Hz, 1H), 8.62 (s, 1H), 8.26 (dd, J = 9.6 Hz, 4.4 Hz, 1H), 8.12 (dd, J = 8.8 Hz, 2.4 Hz, 1H), 8.12 (brs, 1H), 7.81 (s, 1H), 7.79 (d, J = 8.8 Hz, 1H), 7.66 (dd, J = 6.8 Hz, 2.8 Hz, 1H), 7.47 (brs, 1H), 7.30-7.24 (m, 2H), 4.39 (q, J = 7.6 Hz, 2H), 3.71 (s, 3H), 2.80 (d, J = 4.8 Hz, 3H), 1.32 (t, J = 7.2 Hz, 3H); MS (EI) m/z = 464 (M⁺).

### Reference Example 21: 6-((5-Carbamoyl-4-(2-methoxy-3-(methylcarbamoyl)anilino)-2-pyridyl)amino)pyridine-3-carboxylate

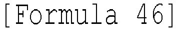

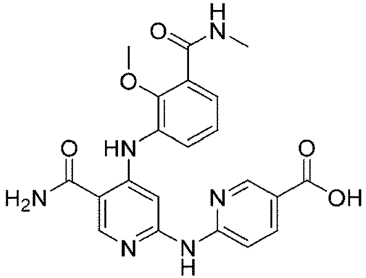

Using ethyl-6-((5-carbamoyl-4-(2-methoxy-3-(methylcarbamoyl)anilino)-2-pyridyl)amino)pyridine-3-carboxylate (Reference Example 20) (0.60 g, 1.29 mmol), the same procedure as in Reference Example 3 was performed to give the title compound (0.43 g, 0.99 mmol, 76% yield).
¹H-NMR (d₆-DMSO 400 MHz) δ 12.9 (brs, 1H), 11.0 (s, 1H), 10.3 (s, 1H), 8.69 (d, J = 2.0 Hz, 1H), 8.62 (s, 1H), 8.29-8.25 (m, 1H), 8.14-8.10 (m, 2H), 7.86 (brs, 1H), 7.72-7.65 (m, 2H), 7.48(brs, 1H), 7.35-7.25 (m, 2H), 3.71 (s, 3H), 2.80 (d, J = 4.8 Hz, 3H); MS (EI) m/z = 436 (M⁺).

### Reference Example 22: Ethyl 10-(6-((5-carbamoyl-4-((2-methoxy-3-(methylcarbamoyl)phenyl)amino)pyridin-2-yl)amino)nicotinamide)decanoate

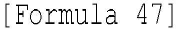

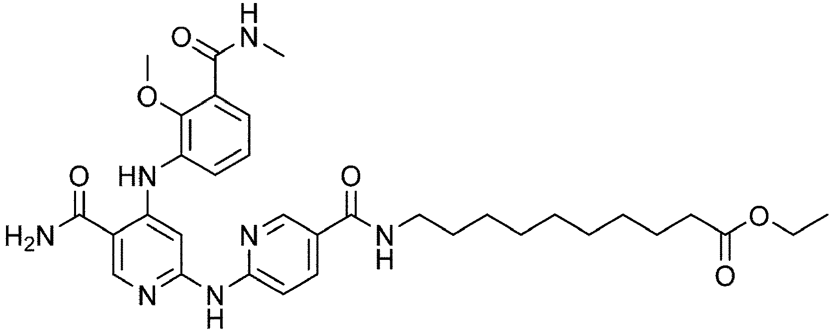

Using 6-((5-carbamoyl-4-(2-methoxy-3-(methylcarbamoyl)anilino)-2-pyridyl)amino)pyridine-3-carboxylate (Reference Example 21) (300 mg, 0.69 mmol) and ethyl 10-aminodecanoate hydrochloride (346 mg, 1.37 mmol), the same procedure as in Reference Example 4 was performed to give the title compound (348 mg, 0.55 mmol, 80 % yield).
¹H-NMR (d₄-MeOH 400 MHz) δ 8.66 (d, J = 2.0 Hz, 1H), 8.52 (s, 1H), 8.06 (dd, J = 8.8 Hz, 2.4 Hz, 1H), 7.73 (dd, J = 8.0 Hz, 1.6 Hz, 1H), 7.73 (brs, 1H), 7.53 (dd, J = 8.0 Hz, 1.6 Hz, 1H), 7.38 (d, J = 8.8 Hz, 1H), 7.31 (t, J = 8.0 Hz, 1H), 4.13-4.07 (m, 2H), 3.79 (s, 3H), 3.36 (t, J = 7.2 Hz, 2H), 2.96 (s, 3H), 2.31-2.25 (m, 2H), 1.68-1.54 (m, 4H), 1.42-1.29 (m, 10H), 1.25-1.19 (m, 3H); MS (EI) m/z = 633 (M⁺).

### Reference Example 23: 10-(6-((5-Carbamoyl-4-((2-methoxy-3-(methylcarbamoyl)phenyl)amino)pyridin-2-yl)amino)nicotinamide)decanoic acid

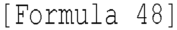

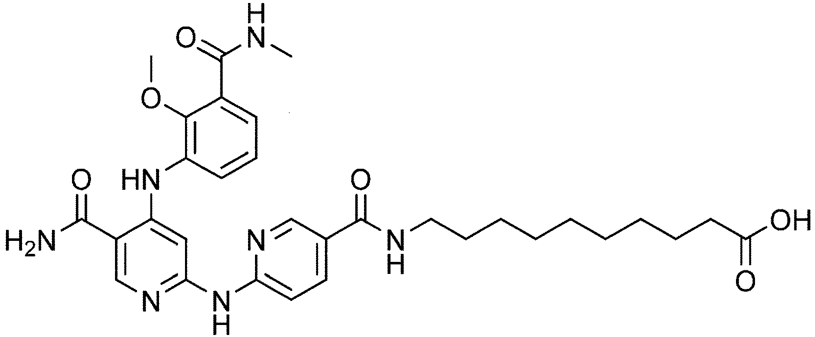

Using ethyl 10-(6-((5-carbamoyl-4-((2-methoxy-3-(methylcarbamoyl)phenyl)amino)pyridin-2-yl)amino)nicotinamide)decanoate (Reference Example 22) (340 mg, 0.54 mmol), the same procedure as in Reference Example 11 was performed to give the title compound (159 mg, 0.26 mmol, 49% yield).
¹H-NMR (d₄-MeOH 400 MHz) δ 8.72 (d, J = 2.0 Hz, 1H), 8.53 (s, 1H), 8.12 (dd, J = 8.8 Hz, 2.4 Hz, 1H), 7.69 (dd, J = 8.0 Hz, 1.6 Hz, 1H), 7.59 (dd, J = 8.0 Hz, 1.6 Hz, 1H), 7.36-7.32 (m, 2H), 7.26 (d, J = 8.8 Hz, 1H), 3.80 (s, 3H), 3.40-3.35 (m, 2H), 2.96 (s, 3H), 2.27 (t, J = 7.6 Hz, 2H), 1.66-1.56 (m, 4H), 1.42-1.29 (m, 10H); MS (EI) m/z = 605 (M⁺).

### Example 9: 6-((5-Carbamoyl-4-((2-methoxy-3-(methylcarbamoyl)phenyl)amino)pyridin-2-yl)amino)-N-(10-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-10-oxodecyl)nicotinamide

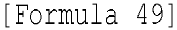

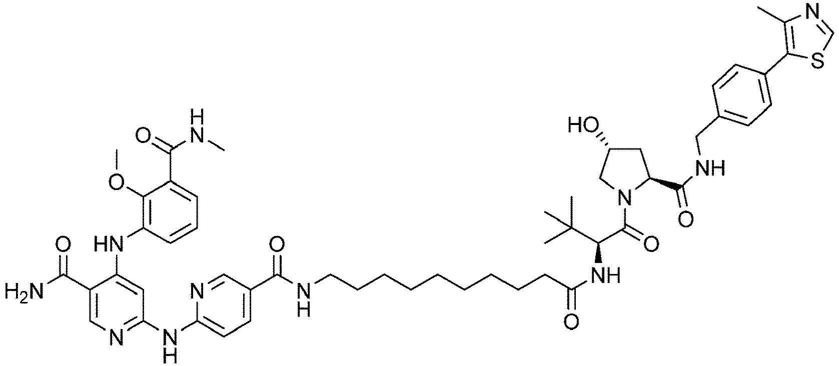

Using 10-(6-((5-carbamoyl-4-((2-methoxy-3-(methylcarbamoyl)phenyl)amino)pyridin-2-yl)amino)nicotinamide)decanoic acid (Reference Example 23) (159 mg, 0.26 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (147 mg, 0.32 mmol), the same procedure as in Example 1 was performed to give the title compound (117 mg, 0.08 mmol, 44% yield).
¹H-NMR (d₄-MeOH 400 MHz) δ 8.86 (s, 1H), 8.65 (d, J = 2.0 Hz, 1H), 8.52 (s, 1H), 8.04 (dd, J = 8.8 Hz, 2.8 Hz, 1H), 7.83 (d, J = 8.8 Hz, 1H), 7.73 (dd, J = 7.6 Hz, 1.6 Hz, 1H), 7.53 (dd, J = 7.6 Hz, 1.6 Hz, 1H), 7.45 (d, J = 8.0 Hz, 2H), 7.42-7.38 (m, 3H), 7.31 (t, J = 7.6 Hz, 1H), 4.64-4.49 (m, 4H), 4.34 (d, J = 15.6 Hz, 1H), 4.34 (d, J = 10.8 Hz, 1H), 3.81-3.78 (m, 1H), 3.79 (s, 3H), 3.36 (t, J = 7.6 Hz, 2H), 2.96 (s, 3H), 2.46 (s, 3H), 2.29-2.18 (m, 3H), 2.10-2.05 (m, 1H), 1.66-1.53 (m, 4H), 1.41-1.27 (m, 10H), 1.03 (s, 9H); MS (ESI) m/z = 1018.5065 (MH⁺).

The synthesis scheme of Example 10 is shown below.

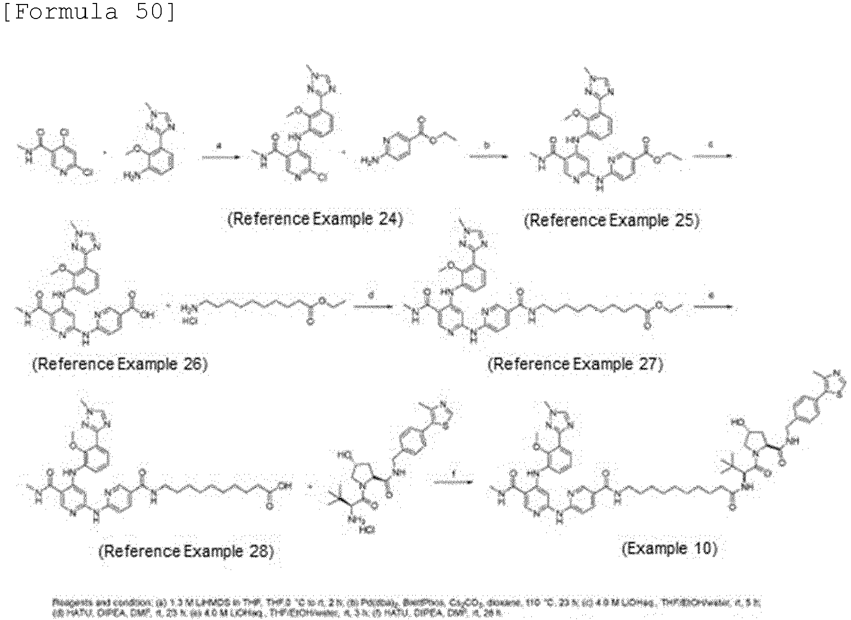

### Synthesis Scheme of Example 10

### Reference Example 24: 6-Chloro-4-((2-methoxy-3-(1-methyl-1-H-1,2,4-triazol-3-yl)phenyl)amino)-N-methylnicotinamide

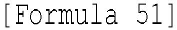

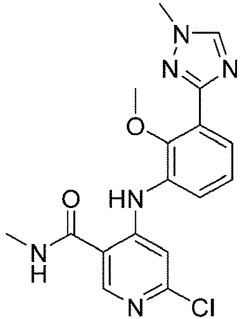

Using 2-methoxy-3-(1-methyl-H-1,2,4-triazol-3-yl)aniline (synthesized according to the method described in J. Med Chem. 2019, 62, 8973.) (0.67 g, 3.28 mmol) and 4,6-dichloro-N-methyl-pyridine-3-carboxamide (0.56 g, 2.73 mmol), the same procedure as in Reference Example 18 was performed to give the title compound (0.79 g, 2.12 mmol, 65% yield).
¹H-NMR (d₄-MeOH, 400 MHz) δ 8.48 (s, 1H), 8.39 (s, 1H), 7.69 (dd, J = 8.0 Hz, 1.6 Hz, 1H), 7.50 (dd, J = 8.0 Hz, 1.6 Hz, 1H), 7.29 (t, J = 8.0 Hz, 1H), 6.99 (s, 1H), 4.02 (s, 3H), 3.71 (s, 3H), 2.92 (s, 3H); MS (EI) m/z = 372 (M⁺).

### Reference Example 25: Ethyl-6-((4-(2-methoxy-3-(1-methyl-1,2,4-triazol-3-yl)anilino)-5-(methylcarbamoyl)-2-pyridyl)amino)pyridine-3-carboxylate

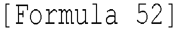

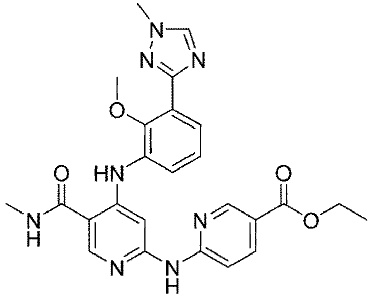

Using 6-chloro-4-((2-methoxy-3-(1-methyl-1-H-1,2,4-triazol-3-yl)phenyl)amino)-N-methylnicotinamide (Reference Example 24) (0.45 g, 1.34 mmol), the same procedure as in Reference Example 2 was performed to give the title compound (0.31 g, 0.61 mmol, 45% yield).
¹H-NMR (d₆-DMSO, 400 MHz) δ 10.7 (s, 1H), 10.3 (s, 1H), 8.70 (d, J = 2.0 Hz, 1H), 8.64 (dd, J = 8.0 Hz, 4.4 Hz, 1H), 8.57 (s, 1H), 8.55 (s, 1H), 8.12 (dd, J = 8.8 Hz, 2.4 Hz, 1H), 7.86 (s, 1H), 7.77 (d, J = 9.2 Hz, 1H), 7.64 (dd, J = 8.4 Hz, 1.2 Hz, 1H), 7.57 (dd, J = 8.0 Hz, 1.6 Hz, 1H), 7.29 (t, J = 8.0 Hz, 1H), 4.30 (q, J = 7.2 Hz, 2H), 3.95 (s, 3H), 3.75 (s, 3H), 2.80 (d, J = 4.4 Hz, 3H), 1.31 (t, J = 7.2 Hz, 3H); MS (EI) m/z = 502 (M⁺).

### Reference Example 26: 6-((4-(2-Methoxy-3-(1-methyl-1,2,4-triazol-3-yl)anilino)-5-(methylcarbamoyl)-2-pyridyl)amino)pyridine-3-carboxylic acid

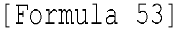

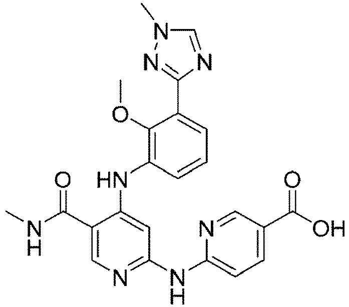

Using ethyl-6-((4-(2-methoxy-3-(1-methyl-1,2,4-triazol-3-yl)anilino)-5-(methylcarbamoyl)-2-pyridyl)amino)pyridine-3-carboxylate (Reference Example 25) (0.14 g, 0.28 mmol), the same procedure as in Reference Example 3 was performed to give the title compound (0.10 g, 0.20 mmol, 72% yield).
¹H-NMR (d₆-DMSO, 400 MHz) δ 12.9 (brs, 1H), 10.7 (s, 1H), 10.2(brs, 1H), 8.69 (d, J = 1.6 Hz, 1H), 8.58-8.57 (m, 1H), 8.57 (s, 1H), 8.52 (s, 1H), 8.10 (dd, J = 8.8 Hz, 2.4 Hz, 1H), 7.93 (brs, 1H), 7.68 (d, J = 8.8 Hz, 1H), 7.65 (dd, J = 8.0 Hz, 1.2 Hz, 1H), 7.58 (d, J = 7.2 Hz, 1H), 7.30 (t, J = 7.6 Hz, 1H), 3.96 (s, 3H), 3.75 (s, 3H), 2.81 (d, J = 4.4 Hz, 1H); MS (EI) m/z = 474 (M⁺).

### Reference Example 27: Ethyl 10-(6-((4-(2-methoxy-3-(1-methyl-1H-1,2,4-triazol 3-yl)phenyl)amino)-5-(methylcarbamoyl)pyridin-2-yl)amino)nicotinamide)decanoate

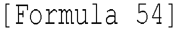

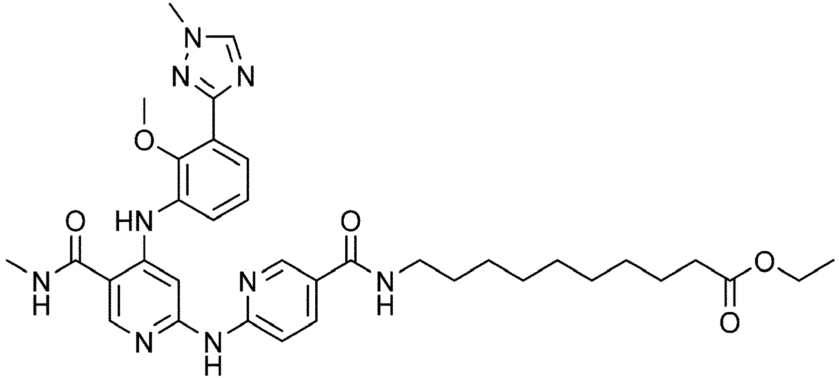

Using 6-((4-(2-methoxy-3-(1-methyl-1,2,4-triazol-3-yl)anilino)-5-(methylcarbamoyl)-2-pyridyl)amino)pyridine-3-carboxylic acid (Reference Example 26) (90 mg, 0.19 mmol) and ethyl 8-aminooctanoate hydrochloride (95.5 mg, 0.38 mmol), the same procedure as in Reference Example 14 was performed to give the title compound (86 mg, 0.13 mmol, 67 % yield).
¹H-NMR (d₄-MeOH 400 MHz) δ 8.65 (d, J = 2.0 Hz, 1H), 8.48 (s, 1H), 8.40 (s, 1H), 8.03 (dd, J = 8.8 Hz, 2.4 Hz, 1H), 7.90 (brs, 1H), 7.71 (dd, J = 8.0 Hz, 1.6 Hz, 1H), 7.59 (dd, J = 8.0 Hz, 1.6 Hz, 1H), 7.45 (d, J = 8.8 Hz, 1H), 7.32 (t, J = 8.0 Hz, 1H), 4.09 (q, J = 7.2 Hz, 2H), 4.02 (s, 3H), 3.75 (s, 3H), 3.36 (t, J = 6.8 Hz, 2H), 2.92 (s, 3H), 2.28 (t, J = 8.0 Hz, 2H), 1.65-1.55 (m, 4H), 1.40-1.29 (m, 10H), 1.22 (t, J = 7.2 Hz, 3H); MS (EI) m/z = 671 (M⁺).

### Reference Example 28: 10-(6-((4-(2-Methoxy-3-(1-methyl-1H-1,2,4-triazol-3-yl)phenyl)amino)-5-(methylcarbamoyl)pyridin-2-yl)amino)nicotinamide)decanoic acid

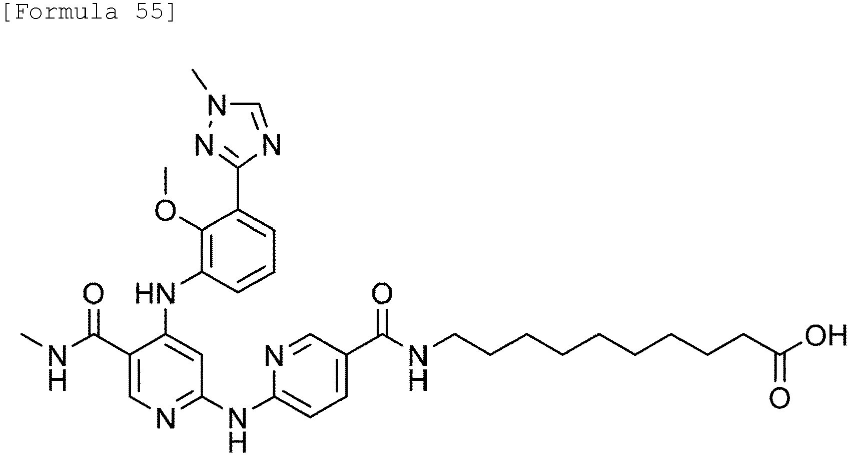

Using ethyl 10-(6-((4-(2-methoxy-3-(1-methyl-1H-1,2,4-triazol-3-yl)phenyl)amino)-5-(methylcarbamoyl)pyridin-2-yl)amino)nicotinamide)decanoate (Reference Example 27) (86.0 mg, 0.13 mmol), the same procedure as in Reference Example 11 was performed to give the title compound (49.0 mg, 0.08 mmol, 59% yield). The thus-obtained title compound was used in the next reaction without further purification.

### Example 10: 6-((5-((10-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-10-oxodecyl)carbamoyl)pyridin-2-yl)amino)-4-((2-methoxy-3-(1-methyl-1H-1,2,4-triazol-3-yl)phenyl)amino)-N-methylnicotinamide

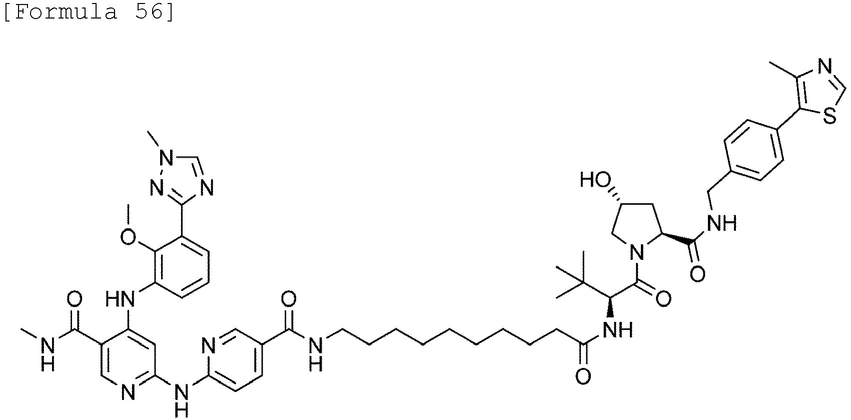

Using 10-(6-((4-(2-methoxy-3-(1-methyl-1H-1,2,4-triazol 3-yl)phenyl)amino)-5-(methylcarbamoyl)pyridin-2-yl)amino)nicotinamide)decanoic acid (Reference Example 28) (49.0 mg, 0.8 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (42.7 mg, 0.09 mmol), the same procedure as in Example 1 was performed to give the title compound (9.50 mg, 0.08 mmol, 12% yield).
¹H-NMR (d₄-MeOH 400 MHz) δ 8.86 (s, 1H), 8.64 (d, J = 2.0 Hz, 1H), 8.47 (s, 1H), 8.39 (s, 1H), 8.02 (dd, J = 8.8 Hz, 2.4 Hz, 1H), 7.84-7.81 (m, 1H), 7.70 (dd, J = 8.0 Hz, 1.6 Hz, 1H), 7.59 (dd, J = 7.6 Hz, 1.6 Hz, 1H), 7.46-7.38 (m, 5H), 7.31 (t, J = 7.6 Hz, 1H), 4.64-4.49 (m, 4H), 4.34 (d, J = 15.2 Hz, 1H), 4.01 (s, 3H), 3.90 (d, J = 10.4 Hz, 1H), 3.81-3.77 (m, 1H), 3.74 (s, 3H), 3.35 (t, J = 7.6 Hz, 2H), 2.92 (s, 3H), 2.46 (s, 3H), 2.29-2.20 (m, 3H), 2.10-2.03 (m, 1H), 1.65-1.54 (m, 4H), 1.40-1.26 (m, 10H), 1.02 (s, 9H) ; MS (ESI) m/z = 1056.3 (MH⁺).

The synthesis scheme of Example 11 is shown below.

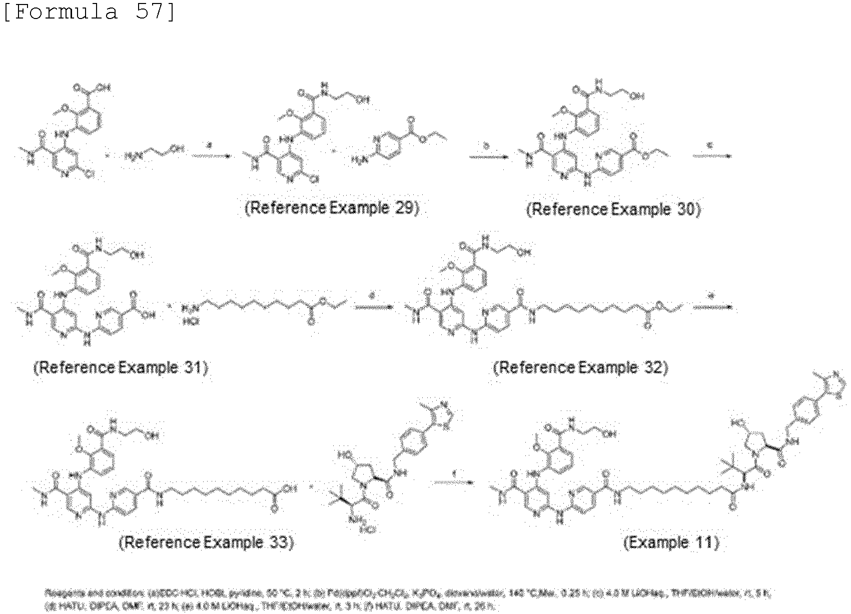

### Synthesis Scheme of Example 1 1

### Reference Example 29: 6-Chloro-4-((3-((2-hydroxyethyl)carbamoyl)-2-methoxyphenyl) amino)-N-methylnicotinamide

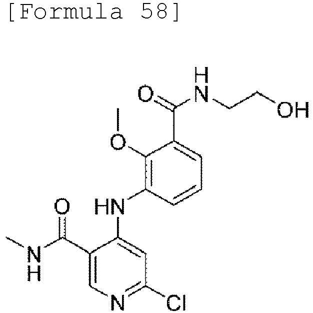

To a 3-((2-chloro-5-(methylcarbamoyl)pyridyl-4-yl)amino)-2-methoxybenzoic acid (synthesized according to the method described in J. Med Chem. 2019, 62, 8973.) (200 mg, 0.60 mmol) solution in pyridine (3.0 mL) was added 2-aminoethanol hydrochloride (116 mg, 1.19 mmol), then 1-(3-dimethylaminopropyl)-3-ethylcarbodiimide hydrochloride (EDCI·HCl) (137 mg, 0.71 mmol) and 1-hydroxybenzotriazole monohydrate (HOBt·H₂O) (18.2 mg, 0.12 mmol). The reaction solution was heated to 50°C, stirred for 2 hours, mixed with chloroform and water, stirred, and then allowed to stand to separate the organic layer. The organic layer was washed twice with water, followed by removal of the solvent under reduced pressure. The thus-obtained residue was purified by silica gel column chromatography (methanol/chloroform = 10%), and the solvent was distilled off under reduced pressure to give the title compound (190 mg, 0.50 mmol, 84% yield).
¹H-NMR (d₆-DMSO, 400 MHz) δ 10.6 (s, 1H), 8.83 (brd, J = 4.8 Hz, 1H), 8.51 (s, 1H), 8.31 (brt, J = 6.0 Hz, 1H), 7.56 (dd, J = 8.0 Hz, 2.0 Hz, 1H), 7.42 (dd, J = 8.0 Hz, 2.0 Hz, 1H), 7.25 (t, J = 8.0 Hz, 1H), 6.90 (s, 1H), 4.76 (t, J = 5.6 Hz, 1H), 3.71 (s, 3H), 3.53 (dd, J =11.6 Hz, 6.0 Hz, 2H), 3.35 (dd, J =11.6 Hz, 6.0 Hz, 2H), 2.81 (d, J = 4.8 Hz, 3H) ; MS (EI) m/z = 378 (M⁺).

### Reference Example 30: Ethyl-6-((4-(3-(2-hydroxyethylcarbamoyl)-2-methoxy-anilino)-5-(methylcarbamoyl)-2-pyridyl)amino)pyridine-3-carboxylate

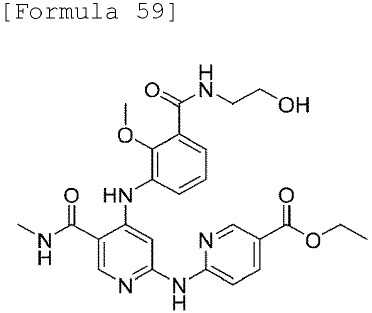

To a 6-chloro-4-((3-((2-hydroxyethyl)carbamoyl)-2-methoxyphenyl)amino)-N-methylnicotinamide (Reference Example 29) (80.0 mg, 0.21 mmol) solution in 1,4-dioxane (0.8 mL) were sequentially added ethyl 6-aminopyridine-3-carboxylate (70.2 mg, 0.42 mmol), (1,1'-bis(diphenylphosphino)ferrocene)dichloropalladium(II) complex with dichloromethane (Pd(dppf)Cl₂·CH₂Cl₂) (34.5 mg, 0.04 mmol), tripotassium phosphate (K₃PO₄) (135 mg, 0.63 mmol), and purified water (0.2 mL), followed by stirring at 140°C for 15 minutes under microwave (MW) irradiation. The reaction solution was allowed to cool to room temperature, then diluted with chloroform, and washed with water, followed by removal of the solvent under reduced pressure. The resulting residue was purified by silica gel column chromatography (methanol/chloroform = 10%) and the solvent was distilled off under reduced pressure to give the title compound (23 mg, 0.05 mmol, 21% yield).
¹H-NMR (CDCl₃, 400 MHz) δ 10.4 (s, 1H), 8.82 (d, J = 2.0 Hz, 1H), 8.31 (s, 1H), 8.28 (brt, J = 6.0 Hz, 1H), 8.16 (dd, J = 8.0 Hz, 2.0 Hz, 1H), 7.89 (dd, J = 8.0 Hz, 2.0 Hz, 1H), 7.71 (s, 1H), 7.67 (dd, J = 8.0 Hz, 1.6 Hz, 1H), 7.48 (brs, 1H), 7.32-7.28 (m, 2H), 6.15-6.14 (m, 1H), 4.38 (q, J = 7.6 Hz, 1H), 3.87 (s, 3H), 3.88-3.51 (m, 2H), 3.67 (dd, J =10.4 Hz, 6.0 Hz, 2H), 3.04 (d, J = 5.2 Hz, 3H), 1.40 (t, J = 7.6 Hz, 3H); MS (EI) m/z = 508 (M⁺).

### Reference Example 31: 6-((4-(3-(2-Hydroxyethylcarbamoyl)-2-methoxy anilino)-5-(methylcarbamoyl)-2-pyridyl)amino)pyridine-3-carboxylic acid

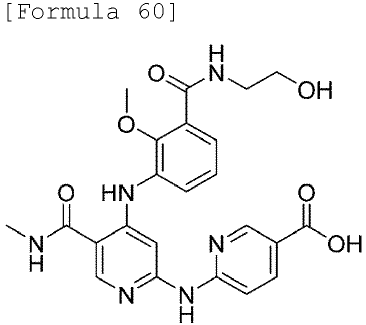

Ethyl-6-((4-(3-(2-hydroxyethylcarbamoyl)-2-methoxy-anilino)-5-(methylcarbamoyl)-2-pyridyl)amino)pyridine-3-carboxylate (Reference Example 30) (23.0 mg, 0.05 mmol) was dissolved in a solvent mixture of ethanol (0.2 mL), tetrahydrofuran (THF) (0.2 mL) and purified water (0.2 mL), then mixed with a 4.0 mol/L lithium hydroxide solution (LiOH) (1.96 mL, 7.83 mmol), followed by stirring overnight at room temperature. The solvent of the reaction solution was distilled off under reduced pressure, and then toluene was added to the residue, followed by further removal of the solvent under reduced pressure to give the title compound. The thus-obtained title compound was used in the next reaction without further purification.

### Reference Example 32: Ethyl 10-(6-((4-((3-((2-hydroxyethyl)carbamoyl)-2-methoxyphenyl)amino)-5-(methylcarbamoyl)pyridin-2-yl)amino)nicotinamide)decanoate

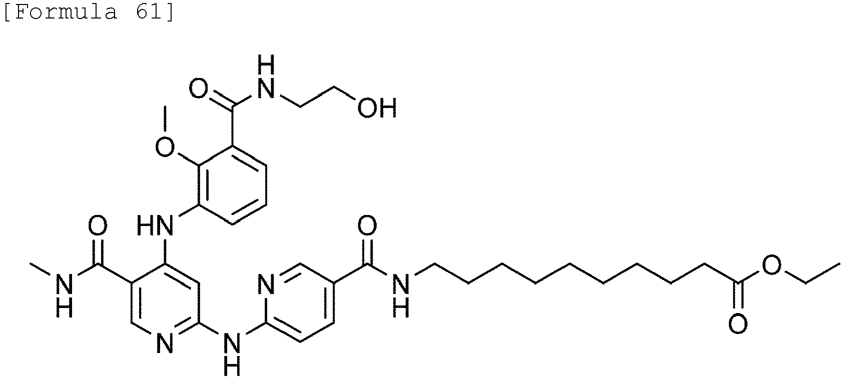

Using 6-((4-(3-(2-hydroxyethylcarbamoyl)-2-methoxy-anilino)-5-(methylcarbamoyl)-2-pyridyl)amino)pyridine-3-carboxylic acid (Reference Example 31) (21.7 mg, 0.05 mmol) and ethyl 10-aminodecanoate hydrochloride (22.7 mg, 0.09 mmol), the same procedure as in Reference Example 4 was performed to give the title compound (21.0 mg, 0.03 mmol, 69 % yield). The thus-obtained title compound was used in the next reaction without performing any structural analysis.

### Reference Example 33: 10-(6-((4-((3-((2-Hydroxyethyl)carbamoyl)-2-methoxyphenyl)amino)-5-(methylcarbamoyl)pyridin-2-yl)amino)nicotinamide)decanoic acid

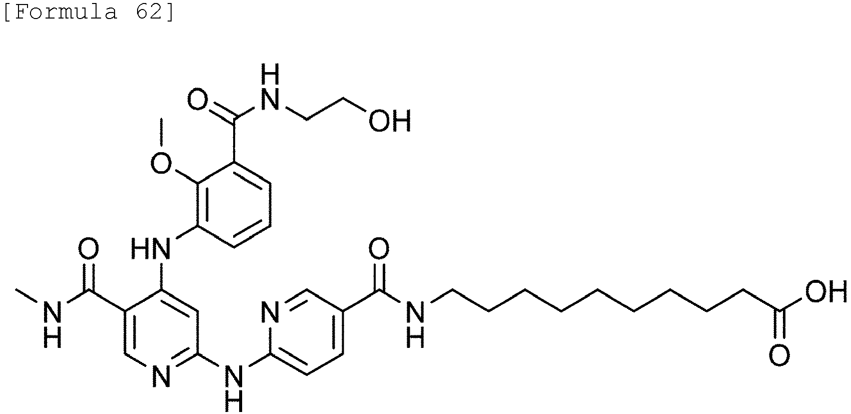

Using ethyl 10-(6-((4-((3-((2-hydroxyethyl)carbamoyl)-2-methoxyphenyl)amino)-5-(methylcarbamoyl)pyridin-2-yl)amino)nicotinamide)decanoate (Reference Example 32) (21.0 mg, 0.03 mmol), the same procedure as in Reference Example 11 was performed to give the title compound (20.1 mg, 0.03 mmol, 100% yield). The thus-obtained title compound was used in the next reaction without further purification.

### Example 11: 6-((5-((10-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-10-oxodecyl)carbamoyl)pyridin-2-yl)amino)-4-((3-((2-hydroxyethyl)carbamoyl)-2-methoxyphenyl)amino)-N-methylnicotinamide

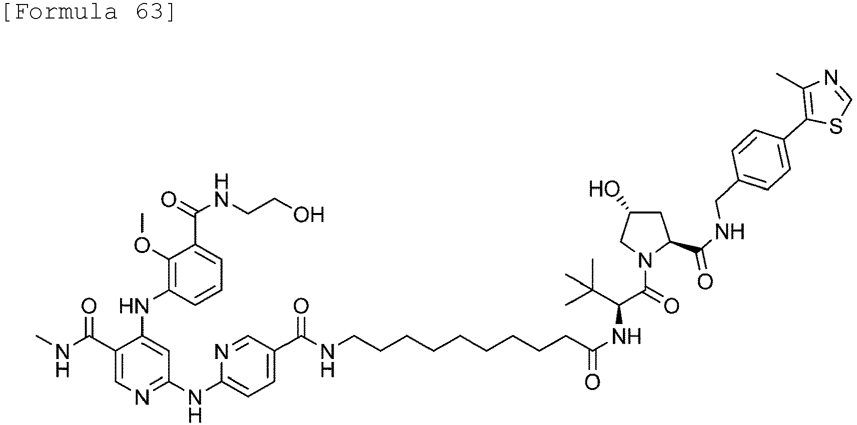

Using 10-(6-((4-((3-((2-hydroxyethyl)carbamoyl)-2-methoxyphenyl)amino)-5-(methylcarbamoyl)pyridin-2-yl)amino)nicotinamide)decanoic acid (Reference Example 33) (20.0 mg, 0.03 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (17.3 mg, 0.04 mmol), the same procedure as in Example 1 was performed to give the title compound (12.3 mg, 0.01 mmol, 38% yield).
¹H-NMR (d₄-MeOH 400 MHz) δ 8.86 (s, 1H), 8.62 (d, J = 1.6 Hz, 1H), 8.40 (s, 1H), 8.02 (dd, J = 8.8 Hz, 2.4 Hz, 1H), 7.80 (d, J = 8.8 Hz, 1H), 7.75 (dd, J = 8.0 Hz, 1.6 Hz, 1H), 7.58 (dd, J = 8.0 Hz, 1.6 Hz, 1H), 7.46-7.42 (m, 3H), 7.39 (d, J = 8.4 Hz, 2H), 7.32 (t, J = 8.0 Hz, 1H), 4.64-4.49 (m, 4H), 4.34 (d, J = 15.6 Hz, 1H), 3.90 (d, J = 11.2 Hz, 1H), 3.84 (s, 3H), 3.81-3.77 (m, 1H), 3.74 (t, J = 5.6 Hz, 2H), 3.56 (t, J = 5.6 Hz, 2H), 3.36 (t, J = 7.2 Hz, 2H), 2.92 (s, 3H), 2.46 (s, 3H), 2.31-2.18 (m, 3H), 2.11-2.05 (m, 1H), 1.66-1.55 (m, 4H), 1.40-1.25 (m, 10H), 1.02 (s, 9H) ; MS (ESI) m/z = 1062.5289 (MH⁺).

The synthesis scheme of Comparative Example 1 is shown below.

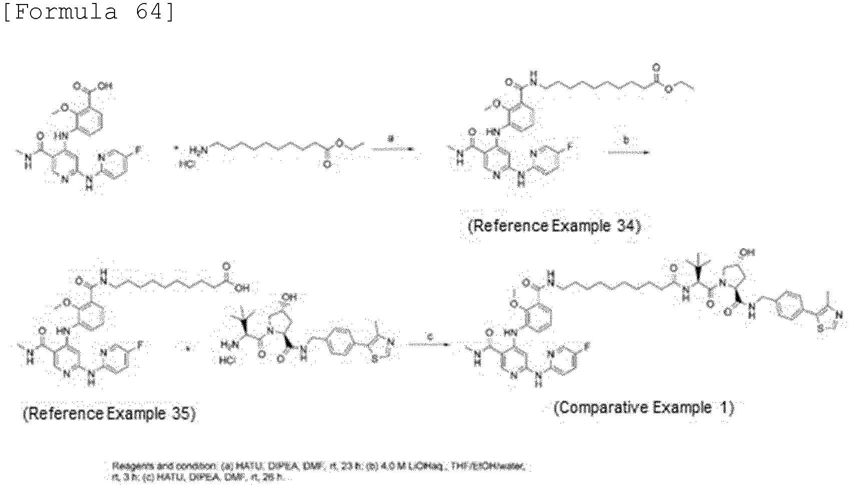

### Synthesis Scheme of Comparative Example 1

### Reference Example 34: Ethyl 10-(3-((2-((5-fluoropyridin-2-yl)amino)-5-(methylcarbamoyl)pyridin-4-yl)amino)-2-methoxybenzamido)decanoate

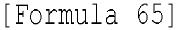

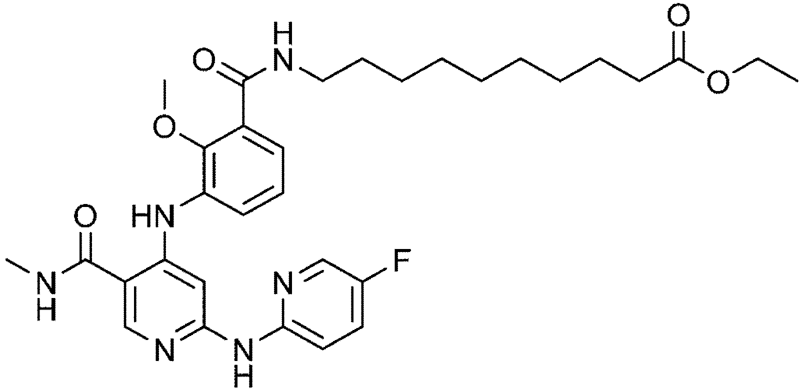

Using 3-((2-((5-fluoropyridin-2-yl)amino)-5-(methylcarbamoyl)pyridin-4-yl)amino)-2-methoxybenzoic acid (synthesized according to the method described in J. Med Chem. 2019, 62, 8973.) (100 mg, 0.24 mmol) and ethyl 10-aminodecanoate hydrochloride (122 mg, 0.49 mmol), the same procedure as in Reference Example 4 was performed to give the title compound (135 mg, 0.22 mmol, 91 % yield). ¹H-NMR (CDCl₃ 400 MHz) δ 10.3 (s, 1H), 8.29 (s, 1H), 8.05 (d, J = 2.4 Hz, 1H), 7.86 (dd, J = 7.6 Hz, 1.6 Hz, 1H), 7.81 (brt, J = 5.2 Hz, 1H), 7.63 (dd, J = 8.0 Hz, 1.6 Hz, 1H), 7.48 (s, 1H), 7.37-7.35 (m, 2H), 7.19 (brs, 1H), 6.13-6.09 (m, 1H), 4.12 (q, J = 7.2 Hz, 2H), 3.83 (s, 3H), 3.70 (q, J = 6.8 Hz, 2H), 3.03 (d J = 5.2 Hz, 3H), 2.28 (t, J = 7.6 Hz, 2H), 1.65-1.57 (m, 4H), 1.42-1.23 (m, 10H), 1.25 (t, J = 7.2 Hz, 3H).

### Reference Example 35: 10-(3-((2-((5-Fluoropyridin-2-yl)amino)-5-(methylcarbamoyl)pyridin-4-yl)amino)-2-methoxybenzamido)decanoic acid

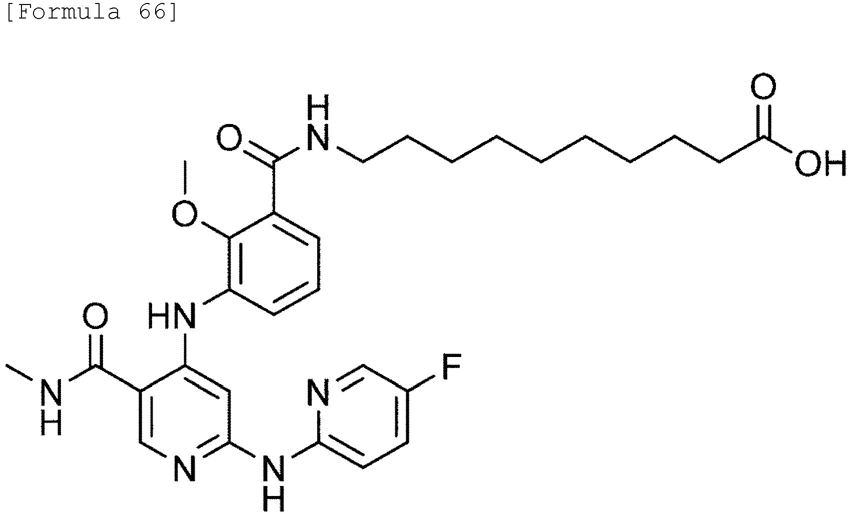

Using ethyl 10-(3-((2-((5-fluoropyridin-2-yl)amino)-5-(methylcarbamoyl)pyridin-4-yl)amino)-2-methoxybenzamido)decanoate (Reference Example 34) (135 mg, 0.27 mmol), the same procedure as in Reference Example 26 was performed to give the title compound (128 mg, 0.22 mmol, 99% yield). The thus-obtained title compound was used in the next reaction without further purification.

### Comparative Example 1: 6-((5-Fluoropyridin-2-yl)amino)-4-((3-((10-(((S)-1-((2S,4R)-4-hydroxy2-((4-(4-methylthiazol-5-yl)benzyl)carbamoyl)pyrrolidin-1-yl)-3,3-dimethyl-1-oxobutan-2-yl)amino)-10-oxodecyl)carbamoyl)-2-methoxyphenyl)amino)-N-methylnicotinamide

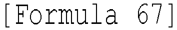

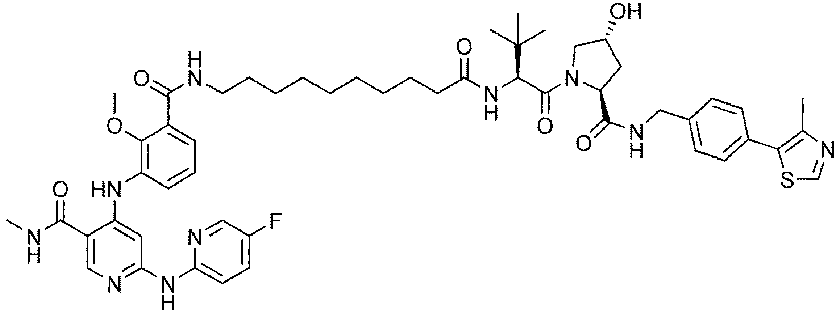

Using 10-(3-((2-((5-fluoropyridin-2-yl)amino)-5-(methylcarbamoyl)pyridin-4-yl)amino)-2-methoxybenzamido)decanoic acid (Reference Example 35) (128 mg, 0.22 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (124 mg, 0.26 mmol), the same procedure as in Example 1 was performed to give the title compound (146 mg, 0.15 mmol, 67% yield).
¹H-NMR (d₄-MeOH 400 MHz) δ 8.86 (s, 1H), 8.35 (s, 1H), 8.19 (d, J = 2.4 Hz, 1H), 7.80 (d, J = 9.2 Hz, 1H), 7.65-7.60 (m, 2H), 7.56 (dd, J = 7.6 Hz, 1.2 Hz, 1H), 7.45 (d, J = 8.4 Hz, 2H), 7.39 (d, J = 8.4 Hz, 2H), 7.32 (t, J = 7.6 Hz, 1H), 7.19 (dd, J = 9.2 Hz, 2.4 Hz, 1H), 4.64-4.49 (m, 4H), 4.34 (d, J = 15.6 Hz, 1H), 3.89 (d, J = 11.2 Hz, 1H), 3.81 (s, 3H), 3.81-3.72 (m, 1H), 3.40 (t, J = 7.2 Hz, 2H), 2.94 (s, 3H), 2.46 (s, 3H), 2.33-2.18 (m, 3H), 2.11-2.04 (m, 1H), 1.67-1.54 (m, 4H), 1.46-1.29 (m, 10H), 1.02 (s, 9H); MS (ESI) m/z = 993.4860 (MH⁺).

The synthesis scheme of Comparative Example 2 is shown below.

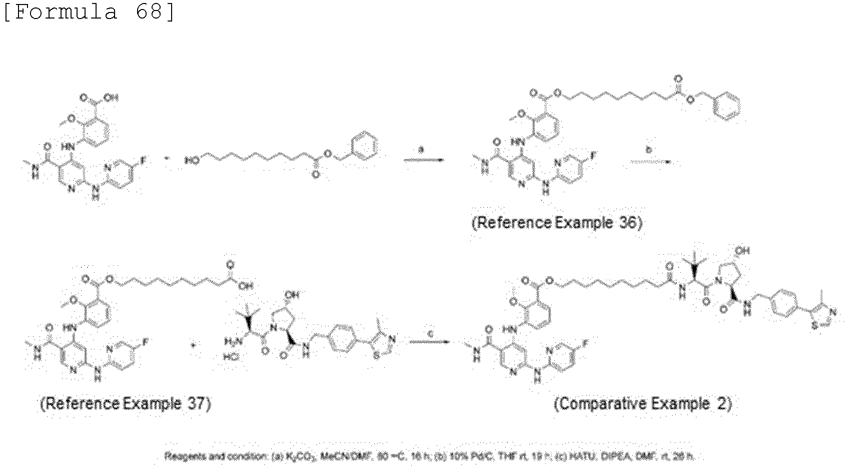

### Synthesis Scheme of Comparative Example 2

### Reference Example 36: 10-(Benzyloxy)-10-oxodecyl 3-((2-((5-fluoropyridin-2-yl)amino)-5-(methylcarbamoyl)pyridin-4-yl)amino)-2-methoxybenzoate

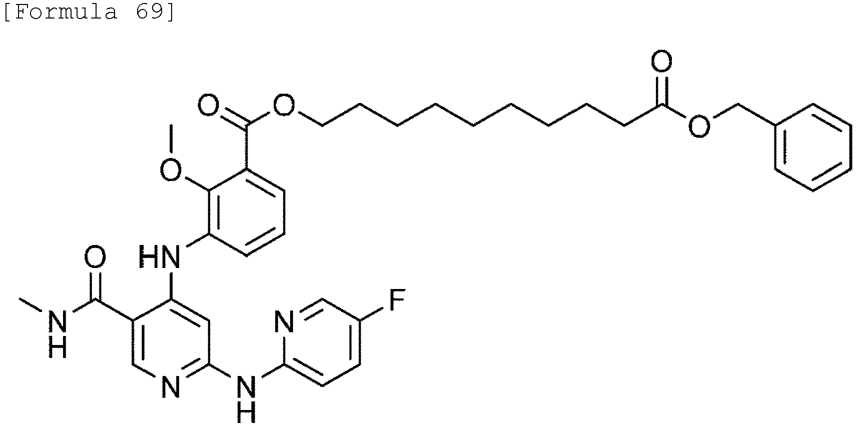

3-((2-((5-Fluoropyridin-2-yl)amino)-5-(methylcarbamoyl)pyridin-4-yl)amino)-2-methoxybenzoic acid (synthesized according to the method described in J. Med Chem. 2019, 62, 8973) (100 mg, 0.24 mmol) was dissolved in a mixed solution of acetonitrile (MeCN) (1.2 mL)/N,N-dimethylformamide (DMF) (1.2 mL), to which benzyl 10-bromodecanoate (124 mg, 0.36 mmol) and potassium carbonate (K₂CO₃) (100 mg, 0.73 mmol) were then added, followed by stirring at 80°C for 16 hours. The resultant was allowed to cool to room temperature, and subjected to filtration to separate the solid. From the thus-obtained filtrate, solvent was distilled off under reduced pressure. The thus-obtained residue was purified by silica gel column chromatography (methanol/chloroform = 10%), and the solvent was distilled off under reduced pressure to give the title compound (94.0 mg, 0.14 mmol, 58 % yield).
¹H-NMR (d₄-DMSO 400 MHz) δ 10.7 (s, 1H), 9.82 (s, 1H), 8.52 (dd, J = 8.8 Hz, 4.4 Hz, 1H), 8.48 (s, 1H), 8.15 (d, J = 3.2 Hz, 1H), 7.76 (dd, J = 8.0 Hz, 1.6 Hz, 1H), 7.72 (s, 1H), 7.70-7.61 (m, 2H), 7.39 (dd, J = 8.0 Hz, 1.6 Hz, 1H), 7.36-7.29 (m, 6H), 5.07 (s, 2H), 4.27 (t, J = 6.4 Hz, 2H), 3.76 (s, 3H), 2.79 (d J = 4.4 Hz, 3H), 2.33 (t, J = 7.6 Hz, 2H), 1.73-1.67 (m, 2H), 1.56-1.50 (m, 2H), 1.42-1.23 (m, 10H) ; MS (EI) m/z = 671(M⁺).

### Reference Example 37: 10-((3-((2-((5-Fluoropyridin-2-yl)amino)-5-((methylcarbamoyl)pyridin-4-yl)amino)-2-methoxybenzoyl)oxy)decanoic acid

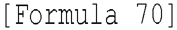

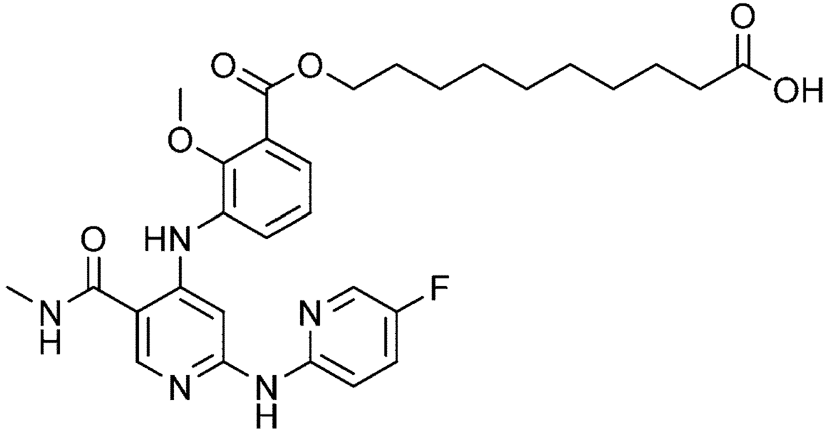

10-(Benzyloxy)-10-oxodecyl 3-((2-((5-fluoropyridin-2-yl)amino)-5-(methylcarbamoyl)pyridin-4-yl)amino)-2-methoxybenzoate (Reference Example 36) (94 mg, 0.14 mmol) was dissolved in tetrahydrofuran (THF) (2 mL), to which 10% palladium/activated carbon catalyst (Pd/C) (20 mg) was then added, followed by stirring under hydrogen atmosphere for 19 hours. The reaction solution was subjected to filtration through filter paper, and the solvent of the filtrate was distilled off under reduced pressure to give the title compound (83.0 mg, 0.14 mmol, 100 % yield).
¹H-NMR (d₄-MeOH 400 MHz) δ 8.36 (s, 1H), 8.07-8.05 (m, 1H), 7.77 (dd, J = 8.0 Hz, 1.6 Hz, 1H), 7.54-7.44 (m, 3H), 7.33-7.25 (m, 2H), 4.33 (t, J = 6.8 Hz, 2H), 3.84 (s, 3H), 2.91 (s, 3H), 2.25 (t, J = 7.2 Hz, 2H), 1.82-1.74 (m, 2H), 1.62-1.55 (m, 2H), 1.52-1.43 (m, 2H), 1.43-1.28 (m, 8H) ; MS (EI) m/z = 581 (M⁺).

### Comparative Example 2: 10-(((S)-1-((2S,4R)-4-Hydroxy 2-((4-(4-methylthiazol-5-yl)benzyl)carbamoyl)pyrrolidin-1-yl)-3,3-dimethyl-1-oxobutan-2-yl)amino)-10-oxodecyl 3-((2-((5-fluoropyridin-2-yl)amino)-5-(methylcarbamoyl)pyridin-4-yl)amino)-2-methoxybenzoate

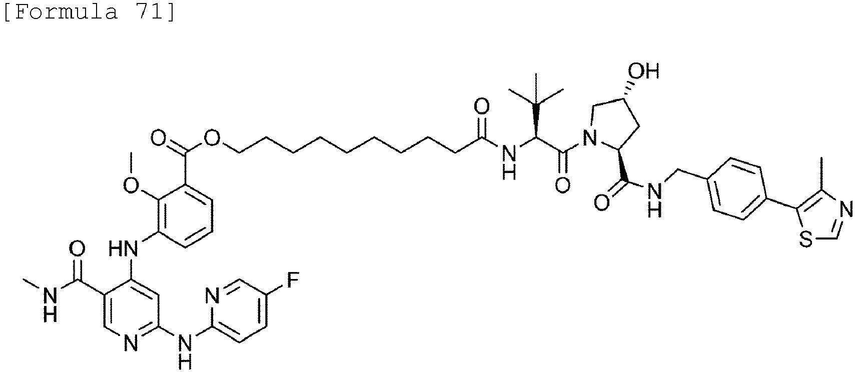

Using 10-((3-((2-((5-fluoropyridin-2-yl)amino)-5-(methylcarbamoyl)pyridin-4-yl)amino)-2-methoxybenzoyl)oxy)decanoic acid (Reference Example 37) (84.1 mg, 0.14 mmol) and (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (synthesized according to the method described in WO2016/118666) (81.1 mg, 0.17 mmol), the same procedure as in Example 1 was performed to give the title compound (69.9 mg, 0.07 mmol, 49% yield).
¹H-NMR (d₄-MeOH 400 MHz) δ 8.85 (s, 1H), 8.36 (s, 1H), 8.04 (d, J = 2.4 Hz, 1H), 7.76 (dd, J = 8.4 Hz, 1.6 Hz, 1H), 7.68 (brs, 1H), 7.50 (dd, J = 8.0 Hz, 1.6 Hz, 1H), 7.47-7.43 (m, 6H), 7.26 (t, J = 7.6 Hz, 1H), 4.64-4.49 (m, 4H), 4.35-4.30 (m, 3H), 3.90 (d, J = 11.2 Hz, 1H), 3.83 (s, 3H), 3.81-3.77 (m, 1H), 2.91 (s, 3H), 2.45 (s, 3H), 2.30-2.18 (m, 3H), 2.11-2.06 (m, 1H), 1.81-1.73 (m, 2H), 1.65-1.53 (m, 2H), 1.51-1.43 (m, 2H), 1.39-1.28 (m, 8H), 1.02 (s, 9H) ; MS (ESI) m/z = 994.4744 (MH⁺).

**[Table 1]**

| Table 1-1 | |
|---|---|
| Exemple compound No. | Structure |
| 1 | 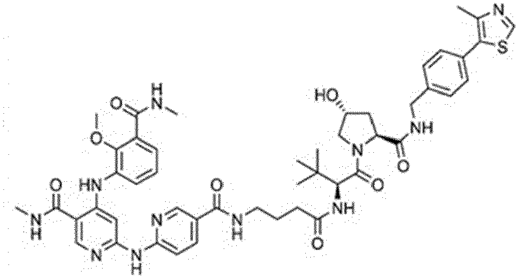 |
| 2 | 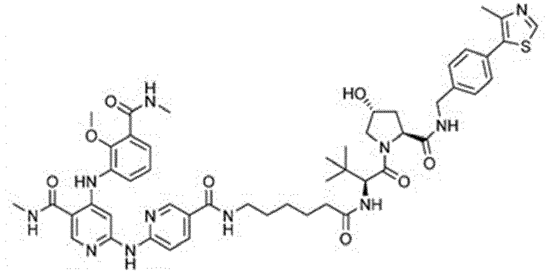 |
| 3 | 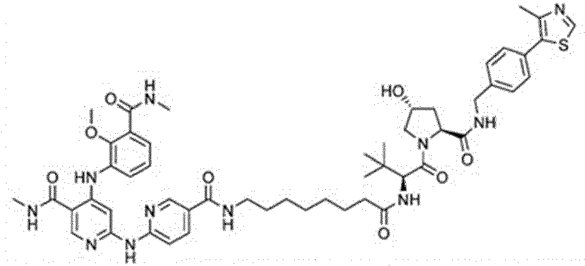 |
| 4 | 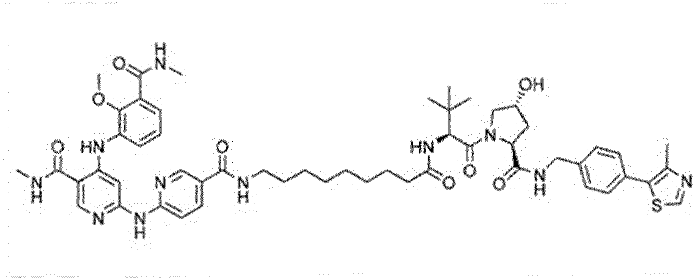 |

**[Table 2]**

| Table 1-2 | |
|---|---|
| Example compound No. | Structure |
| 5 | 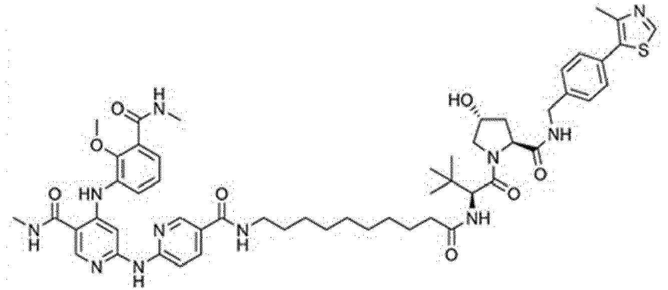 |
| 6 | 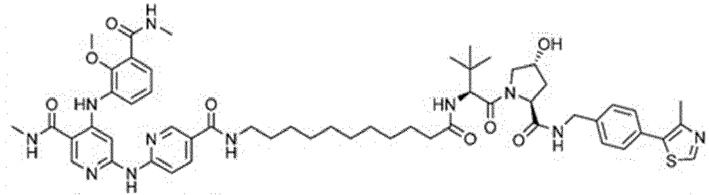 |
| 7 | 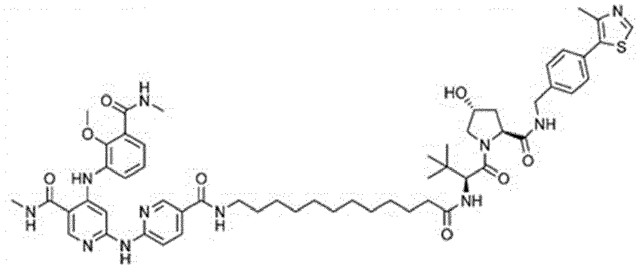 |
| 8 | 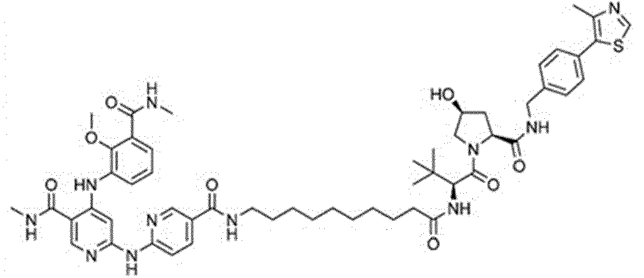 |

**[Table 3]**

| Table 1-3 | |
|---|---|
| Example compound No. | Structure |
| 9 | 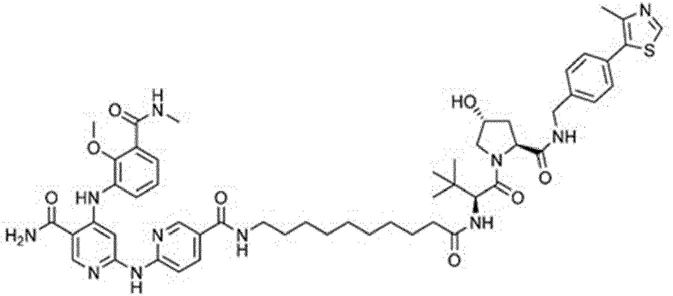 |
| 10 | 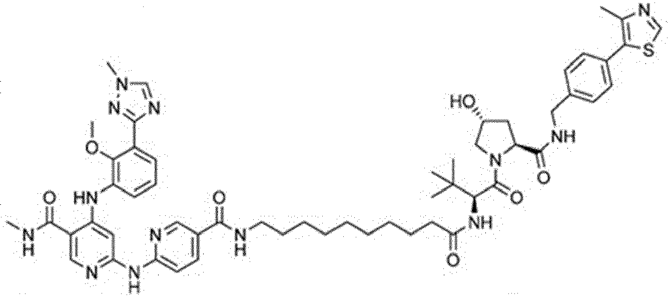 |
| 11 | 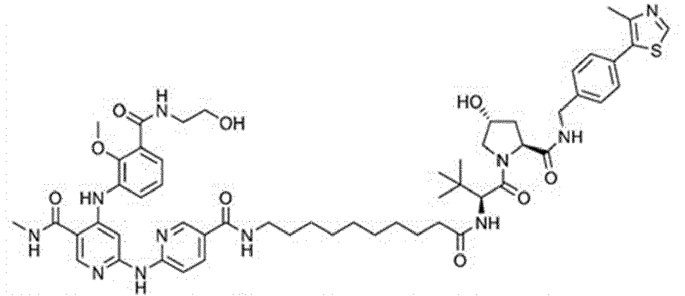 |

**[Table 4]**

| Table 1-4 | |
|---|---|
| Comparative Example compound No. | Structure |
| 1 | 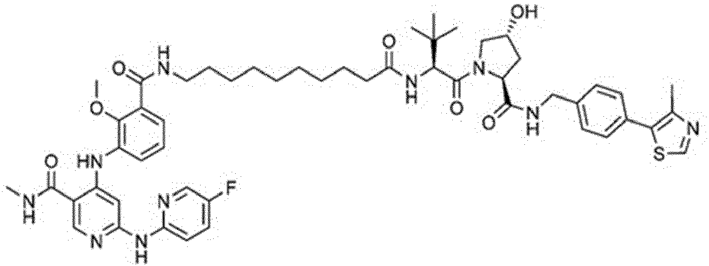 |
| 2 | 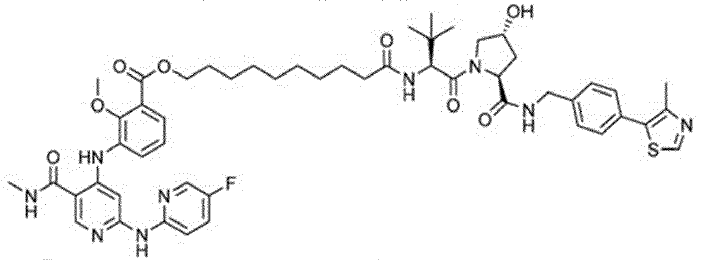 |

### Test Example: Degradation-inducing effect on JAK in Jurkat cells

The degradation-inducing effect of the compound of the present invention on JAK family proteins was evaluated in vitro using Jurkat cells, of which cell line was derived from human leukemic T cells.

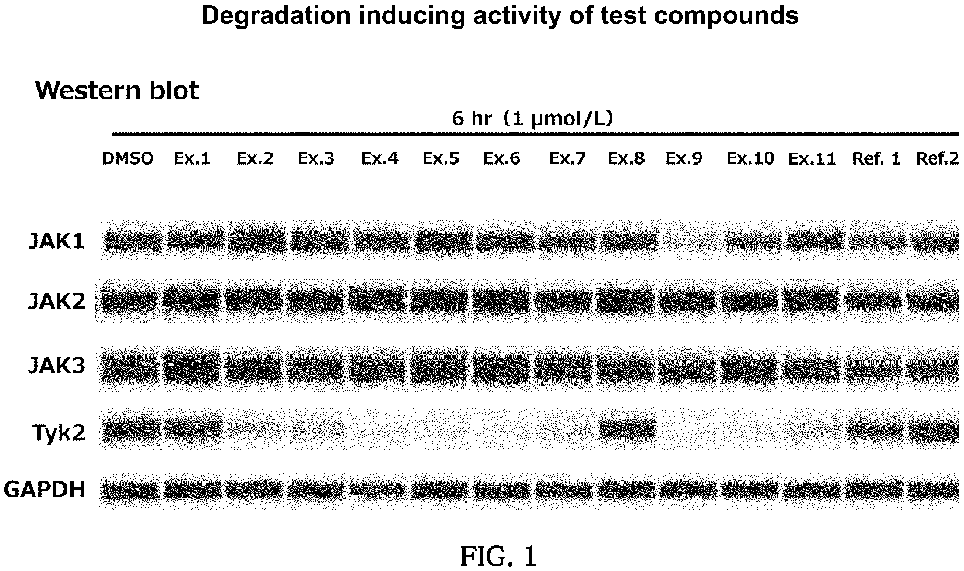

JAK1, JAK2, JAK3, and Tyk2-positive Jurkat cells were seeded at a density of 1 x 10⁵ cells/mL on 10 cm diameter dishes and then cultured in RPMI 1640 medium (FUJIFILM Wako Pure Chemical: catalog number: 189-02025) containing 10% FBS (Thermo Fisher Scientific: catalog number: 10437-028) at 5% CO₂ and 37°C for 3 days. Then, each of the test compounds (compounds of Examples 1 to 11 and Comparative Examples 1 and 2) was added to the dish at a final concentration of 1 µmol/L, and the cells were cultured in the presence of the test compound for 6 hours. After that, the cell culture medium was collected in a 15 mL centrifuge tube (1000 rpm (240 x g), room temperature, 5 min), followed by washing with PBS (1000 rpm (240 x g), room temperature, 5 min). Then, the cells in the centrifuge tube were collected in a 1.5 mL tube (3000 rpm (860 x g), 4°C, 5 min), lysed using RIPA buffer (25 mmol/L Tris-HCl (pH 7.4), 25 mmol/L NaCl, 0.5 mmol/L EGTA (pH 8.0), 0.1% SDS, 1% protease inhibitor), sonicated at ice temperature (cycles of [7.5 sec sonication, 5 sec off] for 5 min) and subjected to centrifugation (15000 rpm (21600 x g), 4°C, 30 min). The thus-obtained supernatant was used as the protein solution. Using the BCA protein assay kit (Thermo Fisher Scientific: catalog number: 23225), the protein concentration of the protein solution of each test compound was measured according to the kit instructions, and then adjusted to 1.4 mg/mL with RIPA buffer. The thus-adjusted protein solutions were subjected to a fully automated Western blotting device (Simple Westerns Wes: proteinSimple) to detect the protein levels (JAK1, JAK2, JAK3, and Tyk2 as the target proteins, and GAPDH (Glyceraldehyde 3-phosphate dehydrogenase) as the internal standard protein) in the protein solutions by chemiluminescent Western blotting. The detection results are shown in Figure 1. The primary antibodies and secondary antibodies used for the detection are shown in Table 5.

**[Table 5]**

| Table 5 List of antibodies | | |
|---|---|---|
| Detection target | Primary antibody | Secondary antibody |
| JAK1 | Anti-JAK1 antibody [EPR349(N)] Rabbit mAb tAbcam, Cat No. ab133666) | Anti-Rabbit Secondary Antibody (proteinsimple, Cat No. 042-206) |
| JAK2 | Jak2 (D2E12) XP Rabbit mAb (Cell Signaling Technology. Cat No. 3230) | Anti-Rabbit Secondary Antibody (proteinsimple. Cat No. 042-206) |
| JAK3 | Jak3 (5H2) Mouse mAb (Cell Signaling Technology, Cat No. 5481) | Anti-Mouse Secondary Antibody (proteinsimple, Cat No. 042-205) |
| Tyk2 | Tyk2 Antibody (Cell Signaling Technology. Cat No. 9312) | Anti-Rabbit Secondary Antibody (proteinsimple. Cat No. 042-206) |
| GAPDH | GAPDH (14C10) Rabbit mAb (Cell Signaling Technology, Cat No. 2118) | Anti-Rabbit Secondary Antibody (proteinsimple. Cat No. 042-206) |

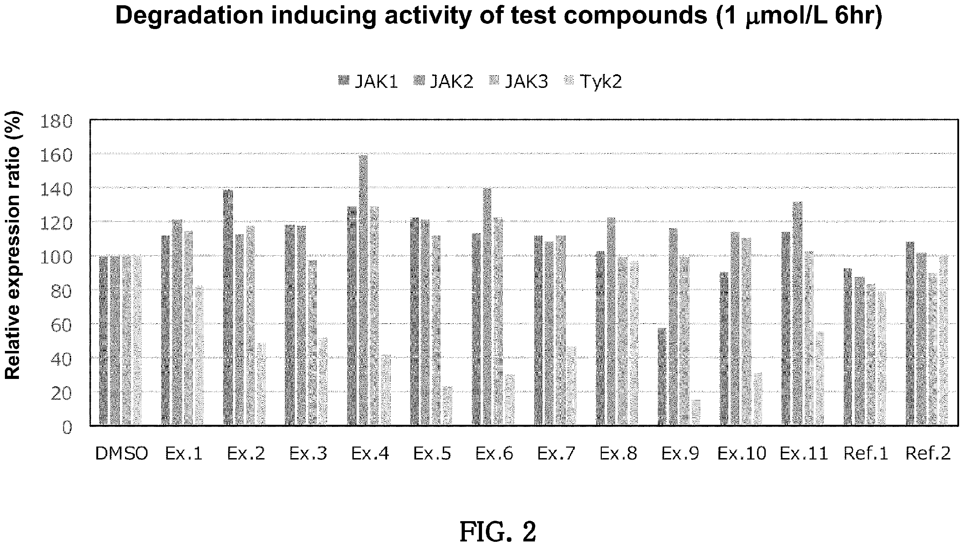

Furthermore, the luminescence levels of JAK1, JAK2, JAK3, and Tyk2 detected by chemiluminescent Western blotting were quantified numerically by using an analysis software (Compass software for Simple Western (version 4.0.0)) attached to the fully automated Western blotting device. The numerical values of the quantified luminescence levels were corrected by the protein levels of the internal standard protein, i.e., GAPDH, (target protein levels/GAPDH protein levels), and compared among the test compounds with dimethyl sulfoxide (DMSO) as 100%. The results are shown in Figure 2 and Table 6.

As is apparent from Figure 2 and Table 6, with regard to the compounds of Examples 1 to 7, 10, and 11, the protein levels of Tyk2 were reduced, while those of JAK1, JAK2, and JAK3 were not reduced, indicating that these compounds have the degradation-inducing activity selectively toward Tyk2. With regard to the compound of Example 9, the protein level of JAK1 was also reduced in addition to that of Tyk2, indicating that the compound of Example 9 has the degradation-inducing activity selectively toward JAK1 and Tyk2. With regard to the compounds of Example 8 and Comparative Examples 1 and 2, the protein levels of JAK1, JAK2, JAK3, and Tyk2 were not reduced, and no degradation-inducing activity toward JAKs was exhibited.

**[Table 6]**

| Table 6 JAK protein ratio of Jurkat cells after treatment of each test compound | | | | | | | | | | | | | | |
|---|---|---|---|---|---|---|---|---|---|---|---|---|---|---|
| compound | DMSO | Example 1 | Example 2 | Example 3 | Example 4 | Example 5 | Example 6 | Example 7 | Example 8 | Example 9 | Example 10 | Example 11 | Comparative Example 1 | Comparative Example 2 |
| JAK1 | 100 | 111.9 | 139.2 | 118.2 | 129.1 | 122.8 | 113.2 | 112.1 | 102.6 | 57.6 | 90.5 | 114.4 | 92.4 | 108.7 |
| JAK2 | 100 | 121.3 | 112.6 | 117.6 | 158.8 | 121.0 | 139.8 | 108.2 | 122.5 | 115.9 | 114.1 | 132.2 | 87.6 | 101.7 |
| JAK3 | 100 | 114.5 | 117.7 | 97.5 | 129.0 | 112.1 | 122.3 | 112.2 | 99.0 | 99.3 | 110.8 | 102.9 | 83.3 | 89.8 |
| Tyk2 | 100 | 82.8 | 48.8 | 51.6 | 41.7 | 23.1 | 30.3 | 47.1 | 97.0 | 15.4 | 31.2 | 55.1 | 78.9 | 100.1 |

### Industrial Applicability

The compound of the present invention has the degradation-inducing activity selectively toward non-receptor tyrosine kinases, especially JAKs, and is capable of modulation, such as the suppression, of activation of cytokine signaling pathways for which JAKs are responsible. Accordingly, the compound of the present invention can be used as a medicament for the prevention or treatment of, for example, autoimmune diseases, inflammatory diseases, COVID-19 infection, cancer, atopic dermatitis, and alopecia, and also as a reagent for research purposes.

## Claims

1. A compound represented by formula I: 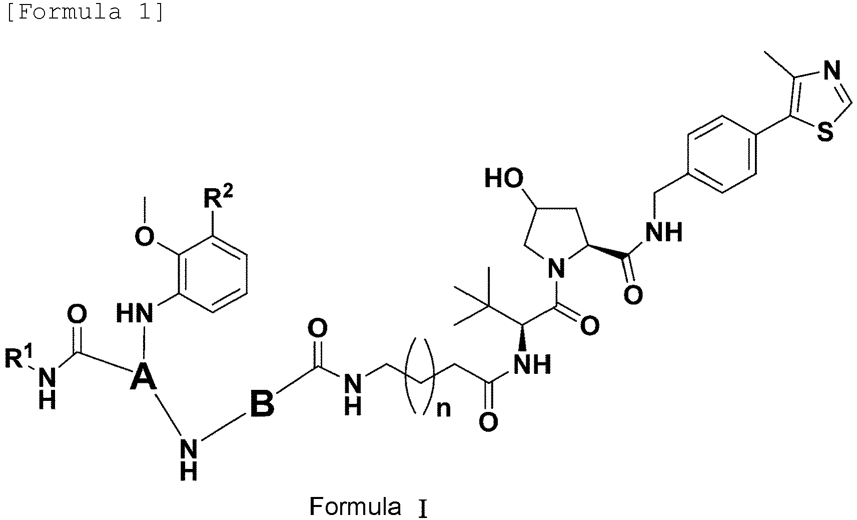 wherein
R¹ is a hydrogen atom or a C₁₋₃ alkyl group optionally substituted with one or more deuterium atoms;
R² is CONHR³ (wherein R³ is a C₁₋₃ alkyl group optionally substituted with one or more OH groups) or a triazole group optionally substituted with one or more C₁₋₃ alkyl groups;
A is a pyridyl group or a pyridazinyl group;
B is 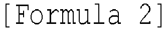 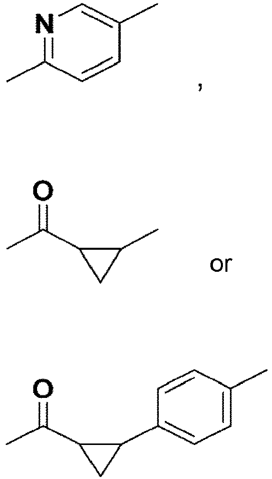 and
n is an integer between 0 and 12,
or a pharmaceutically acceptable salt thereof.

2. The compound according to claim 1 or a pharmaceutically acceptable salt thereof, wherein in formula I, R¹ is a hydrogen atom, a methyl group, or a methyl group substituted with 3 deuterium atoms, and R² is CONHCH₃, CONHCH₂OH, CONHC₂H₅, CONHC₂H₄OH, or a triazole group substituted with one methyl group.

3. The compound according to claim 1 represented by formula II: 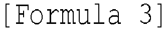 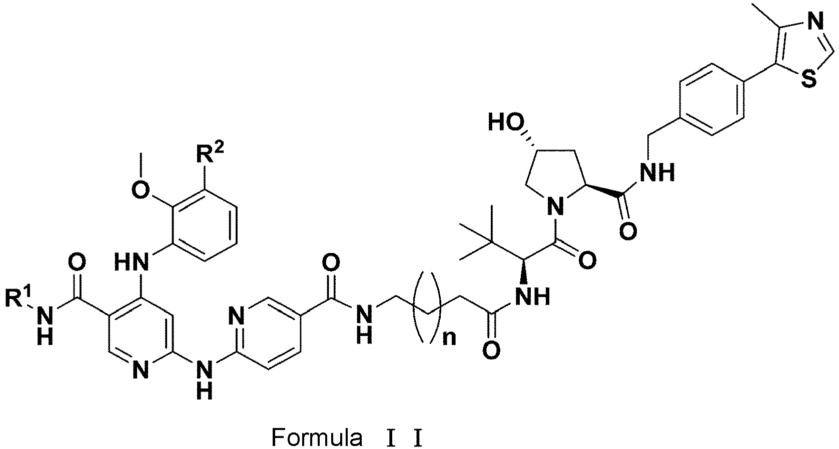 wherein
R¹ is a hydrogen atom, a methyl group, or an ethyl group;
R² is CONHR³ (wherein R³ is as defined in claim 1) or a triazole group optionally substituted with one or more methyl or ethyl groups; and
n is an integer between 1 and 12,
or a pharmaceutically acceptable salt thereof.

4. The compound according to claim 1 represented by formula III: 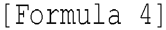 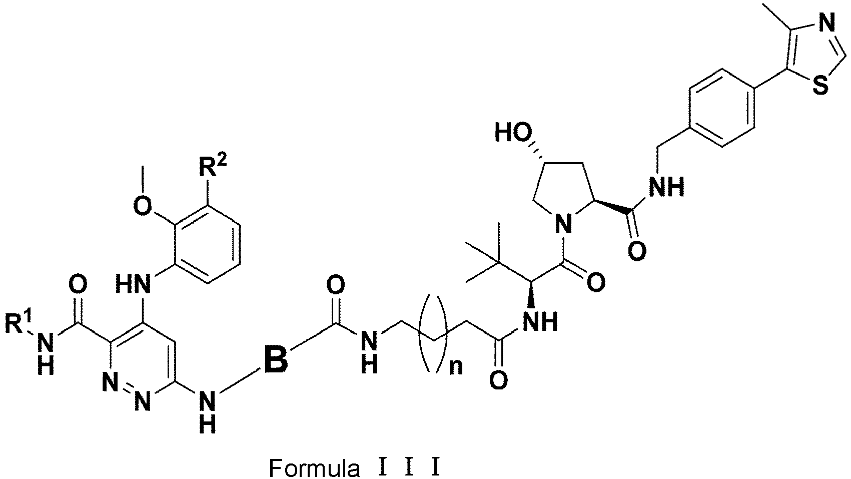 wherein
R¹ is a methyl group substituted with three deuterium atoms;
R² is a triazole group optionally substituted with one or more methyl or ethyl groups;
B is 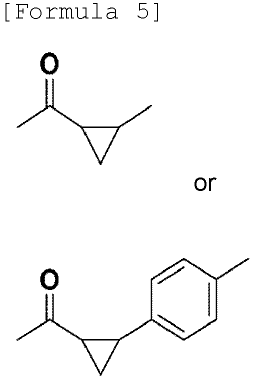
and
n is an integer between 1 and 12,
or a pharmaceutically acceptable salt thereof.

5. A degrader of a non-receptor tyrosine kinase, comprising the compound according to any one of claims 1 to 4 or a pharmaceutically acceptable salt thereof.

6. The degrader according to claim 5, wherein the degrader selectively degrades at least one non-receptor tyrosine kinase selected from the group consisting of JAK1, JAK2, JAK3, and Tyk2.

7. A medicament comprising the compound according to any one of claims 1 to 4 or a pharmaceutically acceptable salt thereof.

8. The medicament according to claim 7, wherein the medicament is used for improving, preventing and/or treating a condition or disease in need of the suppression of activation of the signaling pathway of at least one cytokine selected from the group consisting of IFNα, IFNβ, IFNy, IL-2, IL-4, IL-6, IL-7, IL-9, IL-10, IL-11, IL-12, IL-15, IL-19, IL-20, IL-21, IL-22, IL-23, IL-27, G-CSF, LIF, and oncostatin M.

9. The medicament according to claim 7 or 8, wherein the medicament is a therapeutic agent for preventing or treating an autoimmune disease, an inflammatory disease, COVID-19 infection, cancer, atopic dermatitis, or alopecia.

10. A method of improving, preventing and/or treating a condition or disease requiring the suppression of activation of the signaling pathway of at least one cytokine selected from the group consisting of IFNα, IFNβ, IFNγ, IL-2, IL-4, IL-6, IL-7, IL-9, IL-10, IL-11, IL-12, IL-15, IL-19, IL-20, IL-21, IL-22, IL-23, IL-27, G-CSF, LIF, and oncostatin M, the method comprising administering the medicament according to claim 7 to a subject in need thereof.

11. A use of the compound according to any one of claims 1 to 4 or a pharmaceutically acceptable salt thereof, for producing the medicament according to any one of claims 7 to 9.
